# FASCICULE DE BREVET EUROPEEN

(11) **EP 1 109 817 B1**
(45) Date de publication et mention de la délivrance du brevet: **05.06.2002**
(21) Numéro de dépôt: 99940247.2
(22) Date de dépôt: 27.08.1999
(51) Int. Cl.: C07F 9/09, C07H 19/10, C12N 5/06, A61K 31/66, A61K 49/00

(54) **PHOSPHOHALOHYDRINES, PROCEDE DE FABRICATION ET APPLICATIONS**
PHOSPHOHALOHYDRINE, VERFAHREN ZU DEREN HERSTELLUNG UND VERWENDUNGEN
PHOSPHOHALOHYDRINS, METHOD FOR MAKING SAME AND USES

(30) Priorité: 01.09.1998 FR 9810913
(43) Date de publication de la demande: 27.06.2001
(73) Titulaire: INSTITUT NATIONAL DE LA SANTE ET DE LA RECHERCHE MEDICALE (INSERM), 75013 Paris (FR)
(72) Inventeur: BELMANT, Christian, F-31700 Blagnac (FR); FOURNIE, Jean-Jacques, F-31450 Corronsac (FR); BONNEVILLE, Marc, F-44120 Vertou (FR); PEYRAT, Marie-Alix, F-44230 Saint-Sebastien sur Loire (FR)
(74) Mandataire: Cabinet BARRE LAFORGUE & associés
(86) Numéro de dépôt international: FR9902058
(87) Numéro de publication internationale: WO0012516

(56) Documents cités:
- WO-A-95/20673
- US-A- 5 639 653
- TANAKA Y ET AL: "Natural and synthetic non-peptide antigens recognized by human.gamma..delta. T cells" NATURE (LONDON) (NATUAS,00280836);1995; VOL.375 (6527); PP.155-8, XP002102418 Albert Einstein College Medicine;Howard Hughes Medical Inst.; Bronx; 10461; NY; USA (US)
- WESCH D ET AL: "Comparative analysis of.alpha..beta. and.gamma..delta. T cell activation by Mycobacterium tuberculosis and isopentenyl pyrophosphate" EUR. J. IMMUNOL. (EJIMAF,00142980);1997; VOL.27 (4); PP.952-956, XP002102419 Paul Ehrlich Institute;Department Immunology; Langen; D-63225; Germany (DE)
- BUERK M R ET AL: "Human V.gamma.9-V.delta.2 cells are stimulated in a cross-reactive fashion by a variety of phosphorylated metabolites" EUR. J. IMMUNOL. (EJIMAF,00142980);1995; VOL.25 (7); PP.2052-8, XP002102420 University Hospital;Experimental Immunology, Department of Research; Basel; CH-4031; Switz. (CH)
- SCHOEL B ET AL: "Phosphate is essential for stimulation of V.gamma.9V.delta.2 T lymphocytes by mycobacterial low molecular weight ligand" EUR. J. IMMUNOL. (EJIMAF,00142980);1994; VOL.24 (8); PP.1886-92, XP002102421 University of Ulm;Department of Immunology; Ulm; Germany (DE)
- DHE-PAGANON S ET AL: "MECHANISM OF MEVALONATE PYROPHOSPHATE DECARBOXYLASE: EVIDENCE FOR ACARBOCATIONIC TRANSITION STATE" BIOCHEMISTRY, vol. 33, no. 4, 15 novembre 1994 (1994-11-15), pages 13355-13362, XP000616265

## Description

L'invention concerne de nouveaux composés phosphohalohydrines, leur procédé de fabrication et leurs applications pour la stimulation des lymphocytes Tγ9δ2 porteurs de récepteurs TCR à régions variables Vγ9 et Vδ2.

Les lymphocytes Tγδ des primates présents dans le sang périphérique (humains, singes) représentent, chez l'individu sain, habituellement de 1 à 5% des lymphocytes du sang et jouent un rôle dans le système immunitaire. Il a été démontré qu'ils reconnaissent leurs ligands antigéniques par une interaction directe avec l'antigène, sans présentation par les molécules du CMH d'une cellule présentatrice. Les lymphocytes Tγ9δ2 (parfois aussi désignés lymphocytes Tγ2δ2) sont des lymphocytes Tγδ porteurs de récepteurs TCR à régions variables Vγ9 et Vδ2. Ils représentent la majorité des lymphocytes Tγδ dans le sang humain.

Lorsqu'ils sont activés, les lymphocytes Tγδ exercent une puissante activité cytotoxique non restreinte par le CMH, particulièrement efficace pour tuer divers types de cellules, notamment des cellules pathogènes. II peut s'agir de cellules infectées par des virus ("γδ T cell activation or anergy during infections : the rôle of nonpeptidic TCR ligands and HLA class I molecules" Fabrizio POCCIA *et al*, Journal of Leukocyte Biology, 62, 1997, p. 1-5), ou par d'autres parasites intracellulaires tels que les mycobactéries ("The antituberculous *Mycobacterium bovis* BCG Vaccine is an attenuated Mycobacterial producer of phosphorylated nonpeptidic Antigens for human γδ T cells" Patricia CONSTANT *et al*, Infection and Immunity, vol. 63, n° 12, Dec. 1995, p. 4628-4633) ; ou les protozoaires *("Plasmodium falciparum* stimuli for human γδ T Cells are related to phosphorylated Antigens of mycobacteria" Charlotte BEHR *et al*, Infection and Immunity, Vol. 64, n° 8, 1996, p. 2892-2896). Il peut aussi s'agir de cellules cancéreuses ("CD94/NKG2 inhibitory receptor complex modulates both antiviral and antitumoral responses of polyclonal phosphoantigen-reactive Vγ9 Vδ2 T lymphocytes" Fabrizio POCCIA *et al*, Journal of Immunology, 159, p. 6009-6015 ; "Stimulation of γδ T cells by phosphoantigens" Jean-Jacques FOURNIE, Marc BONNEVILLE, Res. Immunol., 66^{th} FORUM IN IMMUNOLOGY, 147, P. 338-347).

Il a été démontré que les lymphocytes Tγ9δ2 humains réagissent dans le cas d'une infection mycobactérienne à quatre molécules naturelles non peptidiques de structure phosphatée, désignées phosphoantigènes, qui présentent une activité de stimulation pour une concentration de l'ordre de 1 à 5 nM (nanomolaire) (WO-95/20673 et "Stimulation of human γδ T cells by nonpeptidic Mycobacterial ligands" Patricia CONSTANT *et al*, Science, 264, p. 267-270).

Ces antigènes naturels ne sont pas complètement identifiés. Certains auteurs les ont présentés, à tort, comme des dérivés alcènes du pyrophosphate, notamment l'isopentènyl pyrophosphate IPP (US-5 639 653 et "Natural and Synthetic nonpeptide antigens recognized by human γδ T cells", Yoshimasa TANAKA *et al*, Nature, 375, 1995, p. 155-158). Néanmoins, il est maintenant démontré qu'aucun de ces prénylpyrophosphates n'est actif à une concentration de l'ordre du nanomolaire. Les meilleurs résultats obtenus n'arrivent pas à démontrer une activité à moins de 3 µM pour l'IPP, et à 0,3 µM pour le diméthylallyl-UTP et le 3-méthyl-2-hexène pyrophosphate. La concentration minimale d'activité de ces composés est donc, au mieux, de l'ordre de 100 fois plus importante que celle des phosphoantigènes naturels.

En ce qui concerne l'IPP, il est à noter en particulier que les dernières publications mentionnées ci-dessus commettent une erreur en déduisant la structure du radical isopentènyl à partir de la seule analyse du spectre de masse et de la mise en évidence d'une certaine bioactivité. En effet, outre le fait que le composé analysé dans les publications n'était pas purifié et qu'un spectre de masse ne peut pas identifier des espèces non chargées, on peut démontrer qu'il existe en fait plusieurs milliers de structures chimiques différentes pouvant avoir cette même masse moléculaire et être un substituant du pyrophosphate dans ces molécules.

Le fait que la concentration minimale d'activité de l'IPP soit beaucoup plus élevée (de l'ordre de 1000 fois) et que l'intensité des réponses lymphocytaires Tγ9δ2 obtenues soit beaucoup plus faible que celles des phosphoantigènes naturels démontre que l'IPP n'est pas l'un de ces phosphoantigènes naturels ("A novel nucleotide-containing antigen for human blood γδ T lymphocytes", Y. Poquet *et al*, Eur. J. Immunol. 1996, 26, p. 2344-2349). Cela est d'ailleurs confirmé par de nombreuses autres constatations : on ne trouve pas d'IPP en concentration suffisante dans les extraits mycobactériens stimulant les lymphocytes Tγ9δ2 ; l'IPP n'a pas les mêmes caractéristiques chromatographiques (HPAEC) selon : "High pH anion exchange chromatographic analysis of phosphorylated compounds : application to isolation and characterization of non peptide mycobacterial antigens", Y. Poquet *et al,* Anal. Biochem, 243 n° 1, 1996, p. 119-126 que les phosphoantigènes naturels ; l'IPP et les autres isoprénoïdes naturels sont produits par toutes les cellules vivantes, qui ne stimulent pourtant pas les lymphocytes Tγ9δ2.

Par ailleurs, on sait que les substances dont la bioactivité est de l'ordre de ou supérieure à 1 µM ne sont que rarement compatibles avec les contraintes de rentabilité d'une exploitation à l'échelle industrielle. Ainsi, les phosphoantigènes synthétiques proposés jusqu'à maintenant ne sont pas exploitables à l'échelle industrielle dans des conditions économiques acceptables.

Les phosphoantigènes naturels, quant à eux, ne peuvent être produits qu'en très faibles quantités (WO 95/20673), et leur structure chimique exacte restant encore indéterminée, il n'est pas possible de les produire par synthèse. Il n'est donc pas non plus possible d'envisager une exploitation à l'échelle industrielle dans des conditions économiques, malgré leur grand intérêt thérapeutique démontré.

L'invention vise donc à proposer des nouveaux composés chimiques qui soient activateurs des lymphocytes Tγ9δ2 pour une concentration minimale d'activation inférieure à 100nM, notamment de l'ordre de 1 nM.

L'invention vise aussi à proposer des composés pouvant être couplés à un grand nombre de groupements organiques, notamment à des groupements peptidiques naturels ou synthétiques, de façon à permettre l'obtention de composés multifonctionnels.

L'invention vise aussi à proposer de tels composés dont la synthèse est simple, quantitative et peu coûteuse, c'est-à-dire compatible avec les contraintes économiques d'une production à l'échelle industrielle.

L'invention vise aussi à ce titre à proposer une voie de synthèse avantageuse de ces composés.

L'invention vise aussi à proposer un procédé de fabrication de composés selon l'invention.

L'invention vise aussi à proposer des applications des composés selon l'invention à titre d'activateur des lymphocytes Tγ9δ2, et en particulier des applications thérapeutiques des composés selon l'invention.

L'invention conceme donc des composés comprenant au moins un groupement phosphohalohydrine, de formule : 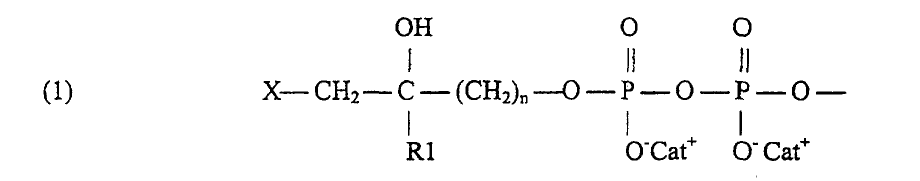 où
X est un halogène choisi parmi l'iode, le brome et le chlore,
R1 est choisi parmi ―CH₃ et ―CH₂―CH₃,
Cat⁺ représente un (ou des) cation(s) organique(s) ou minéral(aux) (y compris le proton) identiques ou différents dans le même composé,
et n est un nombre entier compris entre 2 et 20.

Un composé selon l'invention peut comprendre notamment un ou plusieurs groupement(s) phosphohalohydrine(s) choisi(s) parmi les esters des groupements suivants (nomenclature IUPAC), ou parmi les composés formés de ces groupements :
3-(halométhyl)-3-butanol-1-yl-diphosphate, 3-(halométhyl)-3-pentanol-1-yl-diphosphate, 4-(halométhyl)-4-pentanol-1-yl-diphosphate, 4-(halométhyl)-4-hexanol-1-yl-diphosphate, 5-(halométhyl)-5-hexanol-1-yl-diphosphate, 5-(halométhyl)-4-heptanol-1-yl-diphosphate, 6-(halométhyl)-6-heptanol-1-yl-diphosphate, 6-(halométhyl)-6-octanol-1-yl-diphosphate, 7-(halométhyl)-7-octanol-1-yl-diphosphate, 7-(halométhyl)-7-nonanol-1-yl-diphosphate, 8-(halométhyl)-8-nonanol-1-yl-diphosphate, 8-(halométhyl)-8-décanol-1-yl-diphosphate, 9-(halométhyl)-9-décanol-1-yl-diphosphate, 9-(halométhyl)-9-undécanol-1-yl-diphosphate, 10-(halométhyl)-10-undécanol-1-yl-diphosphate, 10-(halométhyl)-10-dodécanol-1-yl-diphosphate, 11-(halométhyl)-11-dodécanol-1-yl-diphosphate, 11-(halométhyl)-11-tridécanol-1-yl-diphosphate, 12-(halométhyl)-12-tridécanol-1-yl-diphosphate, 12-(halométhyl)-12-tétradécanol-1-yl-diphosphate, 13-(halométhyl)-13-tétradécanol-1-yl -diphosphate, 13-(halométhyl)-13-pentadécanol-1-yl-diphosphate, 14-(halométhyl)-14-pentadécanol-1-yl-diphosphate, 14-(halométhyl)-14-hexadécanol-1-yl-diphosphate, 15-(halométhyl)-15-hexadécanol-1-yl-diphosphate, 15-(halométhyl)-15-heptadécanol-1-yl-diphosphate, 16-(halométhyl)-16-heptadécanol-1-yl-diphosphate, 16-(halométhyl)-16-octadécanol-1-yl-diphosphate, 17-(halométhyl)-17-octadécanol-1-yl-diphosphate, 17-(halométhyl)-17-nonadécanol-1-yl-diphosphate, 18-(halométhyl)-18-nonadécanol-1-yl-diphosphate, 18-(halométhyl)-18-eicosanol-1-yl-diphosphate, 19-(halométhyl)-19-eicosanol-1-yl-diphosphate, 19-(halométhyl)-11-heneicosanol-1-yl-diphosphate,20-(halométhyl)-20-heneicosanol-1-yl-diphosphate, 20-(halométhyl)-20-docosanol-1-yl-diphosphate, 21-(halométhyl)-21-docosanol-1-yl-diphosphate, 21-(halométhyl)-21-tricosanol-1-yl-diphosphate.

Parmi les composés selon l'invention, on peut citer les composés phosphohalohydrines répondant à l'une des formules suivantes : 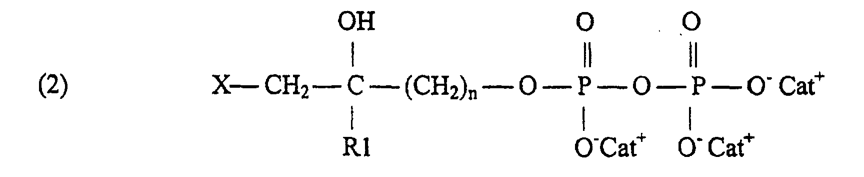 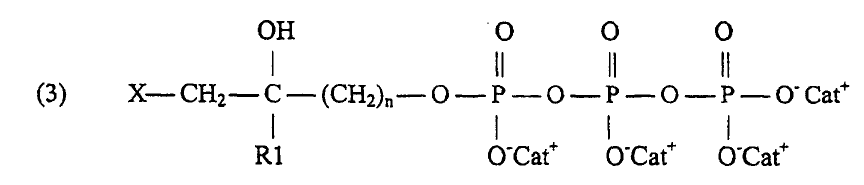 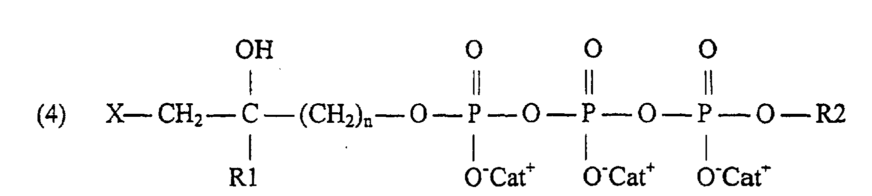 où R2 est un substituant organique ou minéral choisi dans le groupe formé :
- des substituants qui n'empêchent pas la formation de la fonction halohydrine 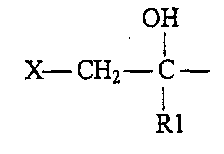 à partir de la fonction alcène 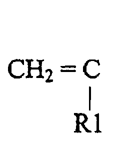 et de l'halogène X₂ en présence d'eau ;
- et des substituants pour lesquels il existe un composé R2-O-Y non réactif sur la fonction halohydrine du composé de formule : 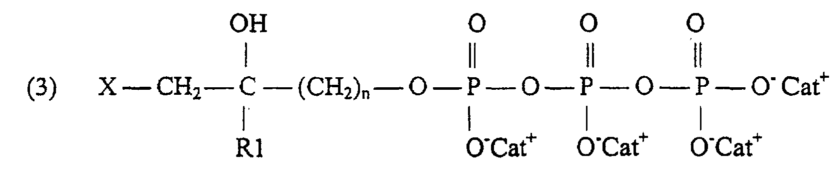 et choisis pour que R2-O-Y puisse réagir sur le phosphate terminal de ce composé (3) pour obtenir le composé (4).

Avantageusement, lesdits composés selon l'invention sont caractérisés en ce que n = 2 et R1 est CH₃.

Les composés selon l'invention comprennent avantageusement en outre au moins un groupement choisi dans le groupe formé des dérivés des nucléosides, des oligonucléotides, des acides nucléiques (ARN, ADN), des acides aminés, des peptides, des protéines, des monosaccharides, des oligosaccharides, des polysaccharides, des acides gras, des lipides simples, des lipides complexes, de l'acide folique, de l'acide tétrahydrofolique, des acides phosphoriques, de l'inositol, des vitamines, des co-enzymes, des flavonoïdes, des aldéhydes, des époxydes et des halohydrines.

En particulier, l'invention s'étend aux composés phosphohalohydrines selon la formule (4) ci-dessus dans lesquels R2 est choisi dans le groupe formé des dérivés des nucléosides, des oligonucléotides, des acides nucléiques (ARN, ADN), des acides aminés, des peptides, des protéines, des monosaccharides, des oligosaccharides, des polysaccharides, des acides gras, des lipides simples, des lipides complexes, de l'acide folique, de l'acide tétrahydrofolique, des acides phosphoriques, de l'inositol, des vitamines, des co-enzymes, des flavonoïdes, des phosphohalohydrines selon la formule (1), des aldéhydes, des époxydes et des halohydrines.

L'invention s'étend aussi aux composés dont la structure incorpore plusieurs groupements conformes à la formule (1), identiques ou différents, par exemple des monomères, polymères, oligomères ou dendrimères, ou plus généralement des molécules à plusieurs branches phosphatées conformes à la formule (1).

Il est à noter que les composés selon l'invention sont des esters (monoesters ou diesters) d'acide phosphorique (ce terme englobant les acides où le phosphore est au degré d'oxydation V, à savoir l'acide orthophosphorique, l'acide pyrophosphorique, l'acide métaphosphorique, l'acide triphosphorique, les autres acides polyphosphoriques).

L'invention s'étend à un procédé de fabrication des composés selon l'invention. Selon l'invention, on fait réagir l'halogène X₂ en présence d'eau avec un composé de départ comprenant au moins un groupement alcène phosphaté de formule :

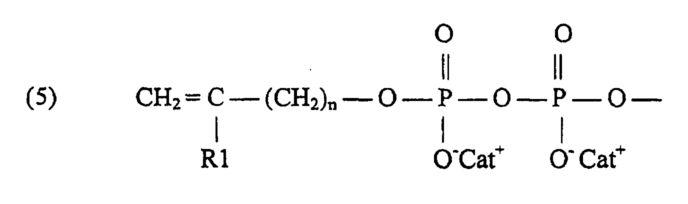

Avantageusement et selon l'invention, on fait réagir un sel formé dudit composé de départ en milieu aqueux ou hydroalcoolique, à pH neutre, à une température inférieure à 30°C, par mélange avec une solution aqueuse de l'halogène X₂. Avantageusement et selon l'invention, on effectue la réaction sous pression atmosphérique à une température comprise entre 0°C et 25°C.
Les composés de départ peuvent être eux-mêmes obtenus à partir de l'alcool :

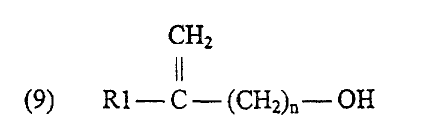

Avantageusement et selon l'invention, le composé de départ est un sel de formule :

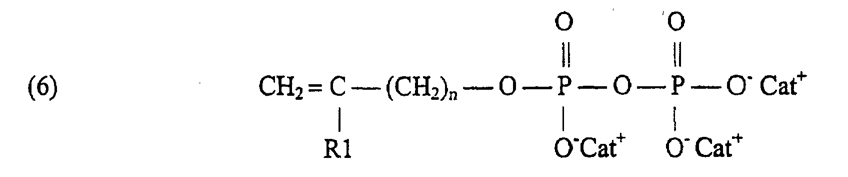

On obtient alors le composé pyrophosphohalohydrine selon la formule (2).

Un exemple de schéma réactionnel complet d'obtention du composé (2) à partir de l'alcool (9) est le suivant : 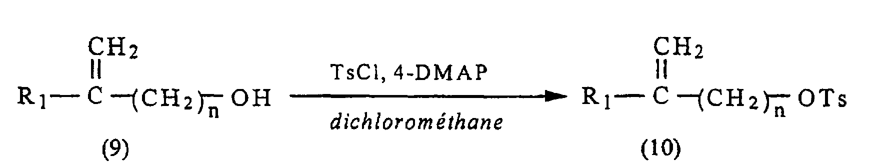 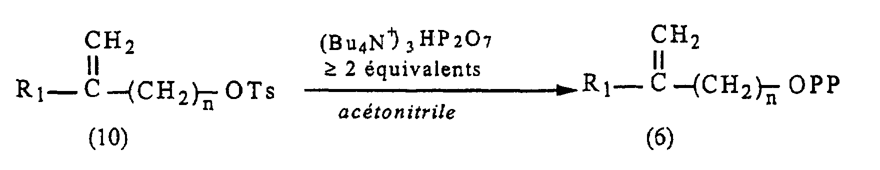 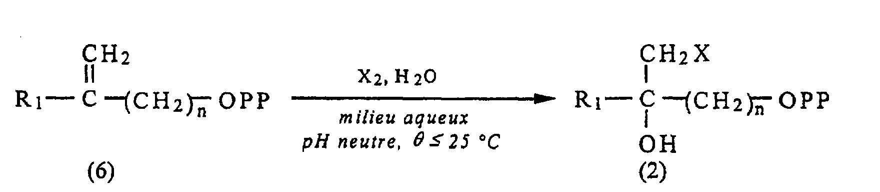
où TsCl est le chlorure de tosyle,
4-DMAP est le 4-diméthylaminopyridine,
Bu₄N+ est le tétrabutylammonium,
(Bu₄N+)₃ HP₂O₇ est le tris (tétra n-butylammonium) hydrogènopyrophosphate,
PP symbolise le groupement pyrophosphate.

Les réactions permettant d'obtenir le composé (6) à partir de l'alcool (9) peuvent être effectuées comme décrit par : DAVISSON V.J. *et al.* "Phosphorylation of Isoprenoid Alcohols" J. Org. Chem 1986, 51,4768-4779, et DAVISSON V.J. *et al.* "Synthesis of Allylic and Homoallylic Isoprenoid Pyrophosphates" Methods in Enzymology, 1984, 110, 130-144.

Avantageusement et selon l'invention, le composé de départ est un sel de formule :

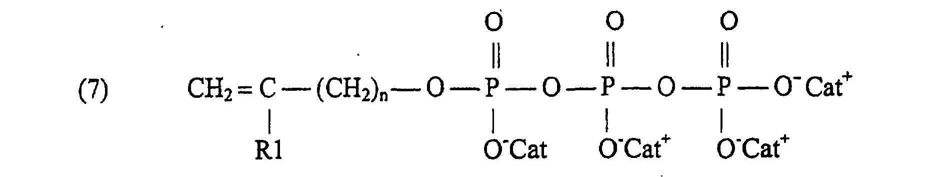

On obtient alors le composé triphosphohalohydrine selon la formule (3).

Un exemple de schéma réactionnel complet d'obtention du composé (3) à partir de l'alcool (9) est le suivant : 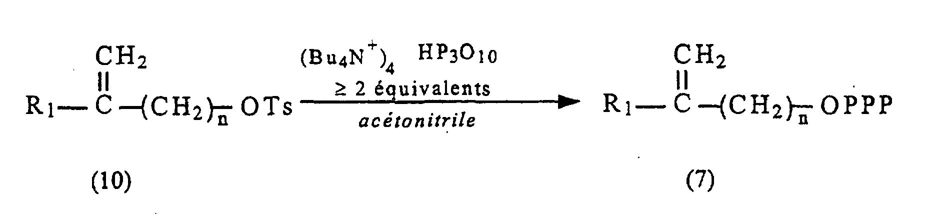 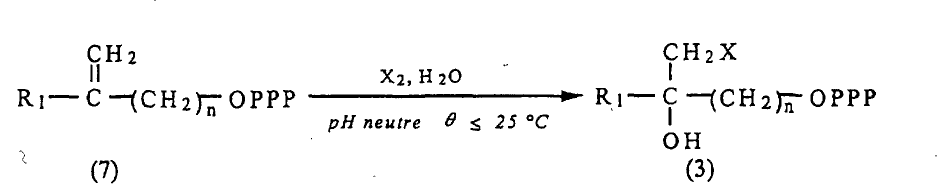
où PPP est le groupement triphosphate,
(Bu ₄N⁺)₄HP₃O₁₀ est le tétrakis (tétra n-butylammonium) hydrogènotriphosphate.

Le composé (10) est obtenu à partir de l'alcool (9) comme indiqué précédemment. La réaction permettant d'obtenir le composé (7) à partir du composé (10) peut être effectuée dans des conditions semblables à celles décrites dans les publications DAVISSON V.J. *et al* ou dans DAVISSON V.J. et *al* "Synthesis of Nucleotide 5'-Diphosphates from (5'-O-Tosyl Nucleosides" J. Org. Chem. 1987, 52, 1794-1801.

Avantageusement et selon l'invention, dans une première variante permettant d'obtenir un composé selon l'invention de formule (4), on peut effectuer le procédé de fabrication selon l'invention mentionné ci-dessus (réaction de X₂ en présence d'eau sur une fonction alcène phosphatée) en prenant, à titre de composé de départ, un sel de formule : 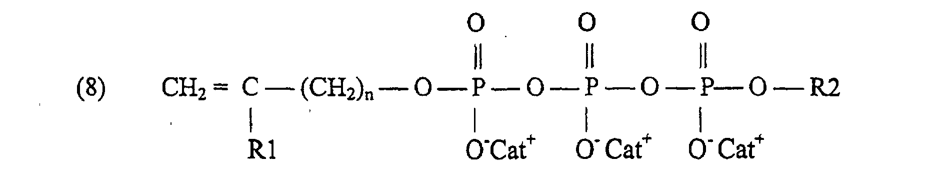 où R2 est un substituant organique ou minéral adapté pour ne pas empêcher la formation de la fonction halohydrine 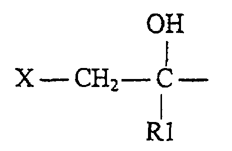 à partir de la fonction alcène 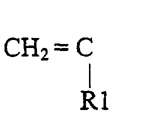 et de l'halogène X₂ en présence d'eau.

Le composé de départ (8) peut lui-même être préparé selon l'un des schémas réactionnels suivants :
- Schéma réactionnel 1 : 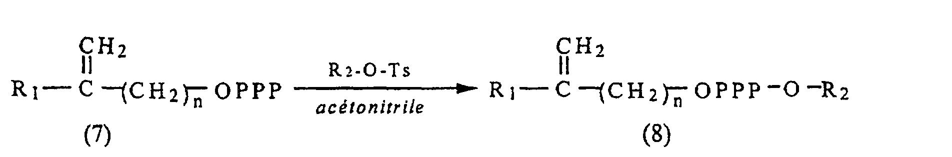
   où Ts est le tosyle.

   Le composé (7) peut être obtenu comme indiqué précédemment à partir de l'alcool (9) et du composé intermédiaire (10). La réaction permettant d'obtenir le composé (8) à partir du composé (7) peut être effectuée dans des conditions semblables à celles décrites dans les publications DAVISSON V.J. *et al.* Ce schéma peut être utilisé lorsque R2-O-Ts est commercialement disponible.
- Schéma réactionnel 2 : 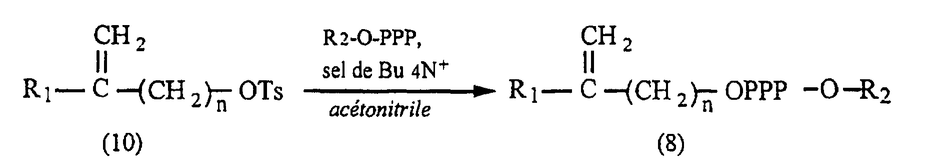
   Le composé intermédiaire (10) peut être obtenu comme indiqué précédemment à partir de l'alcool (9). La réaction permettant d'obtenir le composé (8) à partir du composé (7) peut être effectuée dans des conditions semblables à celles décrites dans les publications DAVISSON V.J. *et al. Ce* schéma peut être utilisé lorsque R2-O-PPP est commercialement disponible.
- Schéma réactionnel 3 : 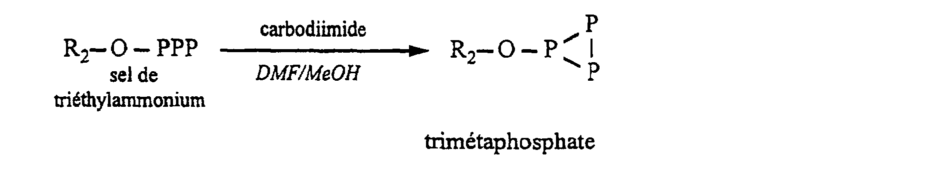 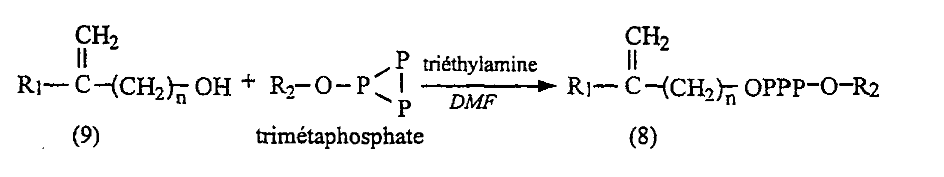
   où DMF est le diméthylformamide,
   MeOH est le méthanol.

Ce schéma réactionnel 3 peut être mis en oeuvre dans des conditions similaires à celles décrites dans D.G. KNORRE *et al* "General method for the synthesis of ATP gamma derivatives" Febs letters, 1976, 70, 105-108.

Ce schéma réactionnel 3 n'est pas utilisable lorsque R2 comporte une fonction réactive au carbodiimide (carboxylate, triphosphate...). Elle est par contre avantageuse lorsque R2-O-PPP est commercialement disponible.

Dans le cas particulier où R2- est lui-même un groupement halohydrine de formule : 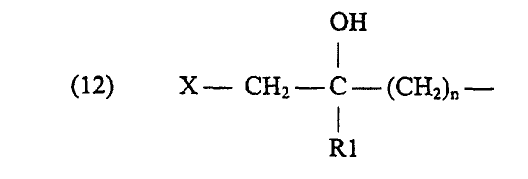 on peut utiliser le schéma réactionnel suivant :

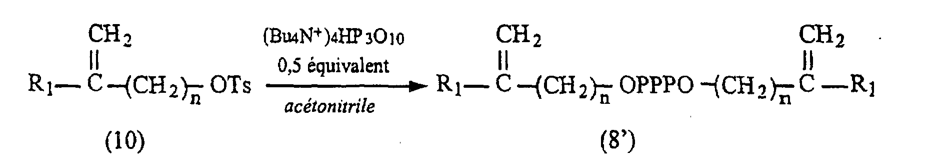

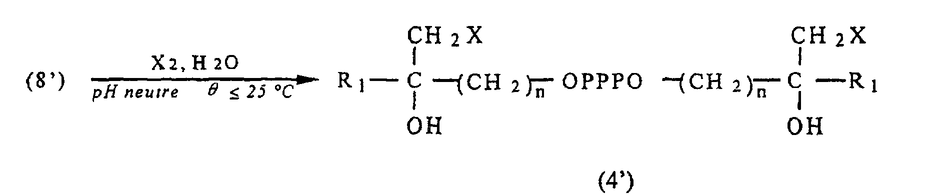

Ce composé (4') est un cas particulier de composé selon l'invention de formule (4).

Il est à noter que dans toutes ces réactions, l'acétonitrile peut être remplacé par tout autre solvant dipolaire aprotique (diméthylformamide DMF, diméthylsulfoxide DMSO...).

Il est à noter qu'à la place du composé intermédiaire (10) pour la préparation des composés (2), (3) et (4'), et dans le cas où n ≠ 2, on peut aussi utiliser le composé chlorure ou bromure correspondant de formule : 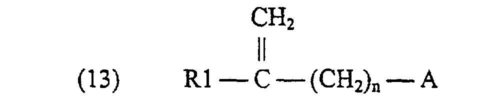
où A est le chlore ou le brome.

Les alcools (9) sont commercialement disponibles ou peuvent être aisément obtenus par une réaction de Grignard bien connue entre un organomagnésien d'alcényle et le formaldéhyde ou l'oxyde d'éthylène.

Dans une deuxième variante applicable dans certains cas, pour préparer un composé selon la formule (4), on pourrait faire réagir le composé triphosphate selon l'invention de formule (3) à partir d'un sel soluble en milieu organique tel qu'un sel de Bu₄N+, dans une étape ultérieure avec un composé R2-O-Y, où -O-Y est un groupement partant et R2 est un substituant organique ou minéral choisis pour que R2-O-Y puisse former, par réaction sur le composé (3), le composé selon l'invention de formule :

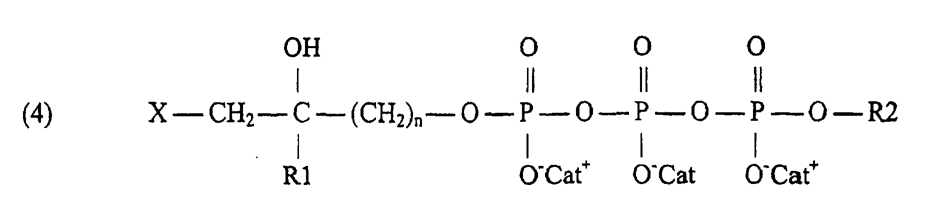

Pour pouvoir former ainsi le composé selon la formule (4), le composé R2-O-Y ne doit notamment pas être réactif sur la fonction halohydrine : 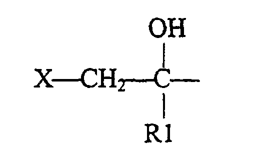 En outre, R2-O-Y doit réagir sur le phosphate terminal du composé (3) pour former le composé (4).

La réaction du composé de formule (3) sur R2-O-Y est une substitution nucléophile. Cette réaction est en particulier possible et avantageuse pour R2 choisi dans le groupe formé des alkyles et des alcènes. Y est choisi de telle sorte que R2-O-Y puisse donner le composé (4) par substitution nucléophile. Y est par exemple choisi parmi le tosyle, le brosyle et le triflyle.

Ainsi, un composé selon l'invention peut être bifonctionnel ou multifonctionnel. La(les) fonction(s) phosphohalohydrine(s) procure(nt) une propriété antigénique spécifique recherchée vis-à-vis des lymphocytes Tγ9δ2, et R2 ou les autres groupements fonctionnels du composé peuvent présenter d'autres propriétés, notamment thérapeutiques.

Dans le cas d'un composé selon l'invention ayant plusieurs groupements phosphohalohydrines conformes à la formule (1), il suffit soit de partir d'un composé de départ ayant le nombre correspondant de groupements alcènes phosphatés de formule (5) et la structure chimique correspondante, soit d'utiliser le composé de formule (3) et de le faire réagir avec un composé intermédiaire R2-O-Y ayant le nombre correspondant de fonctions -O-Y.

L'invention concerne aussi en particulier les nouveaux composés β-esters de phosphohalohydrines de formule : 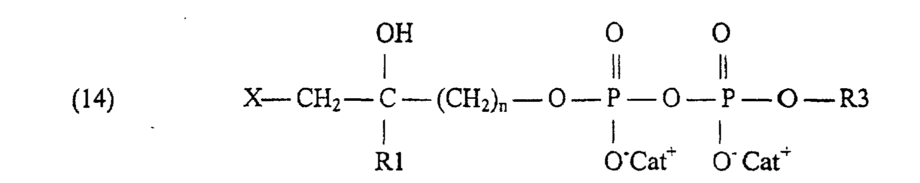 où
X est un halogène choisi parmi l'iode, le brome et le chlore,
R1 est choisi parmi ―CH₃ et ―CH₂―CH₃,
Cat⁺ représente un (ou des) cation(s) organique(s) ou minéral(aux) (y compris le proton) identiques ou différents dans le même composé,
n est un nombre entier compris entre 2 et 20,
R3- est choisi parmi :
   - un groupement halohydrine de formule (12),
   - un groupement époxyde de formule : 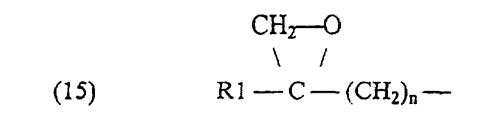
   - un groupement alcène de formule : 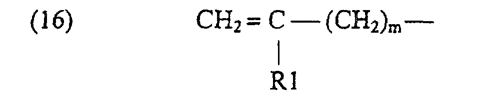 m étant un nombre entier compris entre 1 et 20.

Pour obtenir ces composés (14), on réalise tout d'abord l'étape initiale suivante :

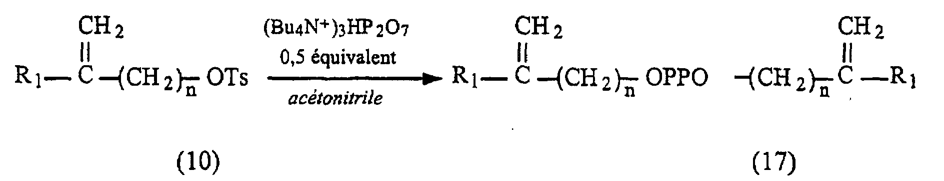

Ensuite, pour obtenir le composé symétrique (14a) diphosphodihalohydrine, on procède de la façon suivante :

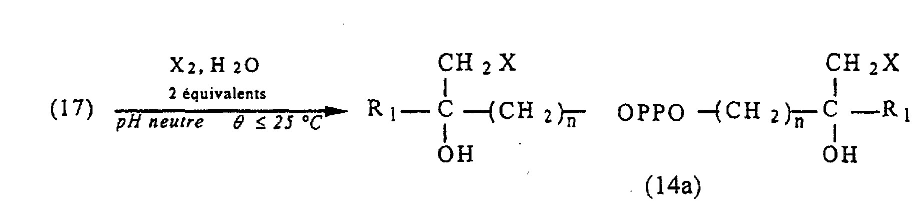

Pour obtenir le composé α,β phosphodiester halohydrine-alcène asymétrique (14b), on procède de la façon suivante :

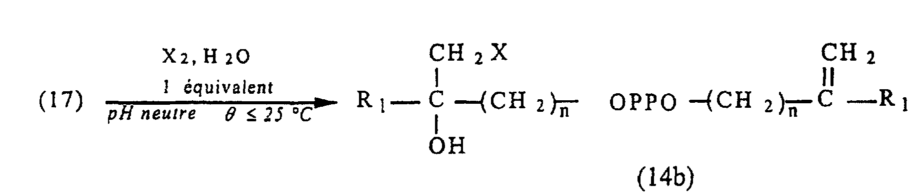

Pour obtenir le composé α,β phosphodiester halohydrine-époxide asymétrique (14c), on procède de la façon suivante :

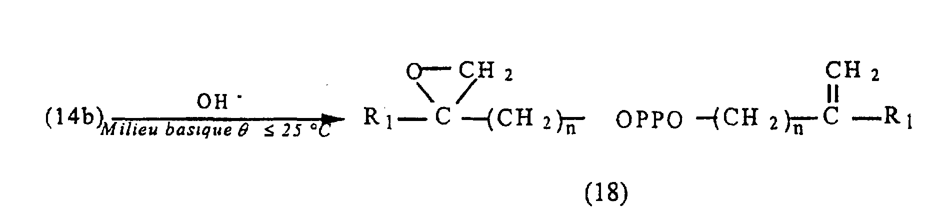

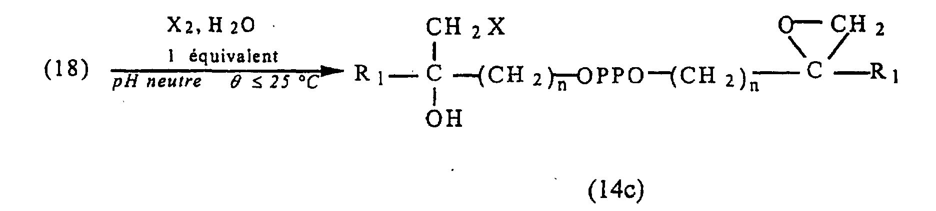

L'invention s'étend également aux utilisations des composés selon l'invention -notamment les composés selon la formule (2)- à titre d'activateurs des lymphocytes Tγ9δ2 des primates, notamment à titre d'activateur de la prolifération et/ou de l'activité cytotoxique et/ou de la production de substance(s) médiatrice(s) des lymphocytes Tγ9δ2 des primates à récepteurs TCR comprenant les régions variables Vγ9 et Vδ2.

L'invention s'étend aussi aux applications des composés selon l'invention pour le traitement de cellules sensibles aux lymphocytes Tγ9δ2 des primates, dans un milieu naturel ou artificiel pouvant contenir des lymphocytes Tγ9δ2, dans lequel lesdites cellules peuvent être mises en contact avec ces lymphocytes Tγ9δ2, et qui est compatible avec les composés selon l'invention (c'est-à-dire n'est pas susceptible d'en provoquer la dégradation au moins dans certaines conditions du traitement).

Par "cellule sensible aux lymphocytes Tγ9δ2", on entend toute cellule sujette à l'activité effectrice induite des lymphocytes Tγ9δ2 (mort cellulaire (destruction cellulaire par les lymphocytes Tγ9δ2); réception de cytokine relarguée par les lymphocytes Tγ9δ2 (TNF-α, INF-γ...); éventuellement prolifération cellulaire induite par les lymphocytes Tγ9δ2.

L'invention s'étend donc à un procédé d'activation des lymphocytes Tγ9δ2 -notamment à un procédé d'activation de la prolifération des lymphocytes Tγ9δ2 et/ou de l'activité cytotoxique des lymphocytes Tγ9δ2 et/ou de la production de substance(s) médiatrice(s) par les lymphocytes Tγ9δ2- dans lequel on met ces lymphocytes Tγ9δ2 au contact d'au moins un composé selon l'invention dans un milieu contenant des lymphocytes Tγ9δ2 compatible avec la croissance lymphocytaire T. Avantageusement et selon l'invention, on introduit dans le milieu une proportion d'interleukine -notamment d'interleukine 2 (IL2)-adaptée pour engendrer une croissance lymphocytaire dans le milieu. En effet, la présence du facteur de croissance lymphocytaire IL2 est indispensable pour obtenir la prolifération des lymphocytes T parmi lesquels seuls les lymphocytes Tγ9δ2 ont été activés par un composé selon l'invention. Ainsi, ce facteur de croissance doit être présent dans le milieu pour les applications où l'on recherche une prolifération des lymphocytes Tγ9δ2. Ce facteur de croissance lymphocytaire peut préexister à l'état naturel, ou être induit ou introduit dans le milieu, simultanément ou non à l'incorporation du composé selon l'invention, dans la même composition thérapeutique ou non. Néanmoins, pour certaines applications où une activation sans prolifération des lymphocytes Tγ9δ2 est recherchée (par exemple la cytotoxicité induite), la présence de ce facteur de croissance n'est pas utile.

Plus particulièrement, l'invention s'étend aux applications des composés selon l'invention à titre thérapeutique pour le traitement curatif ou préventif des pathologies produisant des cellules sensibles aux lymphocytes Tγ9δ2 des primates dans un milieu pouvant contenir ces lymphocytes Tγ9δ2 et dans lequel ces cellules peuvent être mises au contact des lymphocytes Tγ9δ2.

Avantageusement et selon l'invention, on utilise au moins un composé selon l'invention à une concentration dans le milieu qui procure une activation de la prolifération polyclonale des lymphocytes Tγ9δ2. Ce milieu peut être choisi parmi le sang humain, le sang d'un primate non humain, les extraits de sang humain, et les extraits de sang d'un primate non humain.

Ledit milieu peut être extracorporel, ledit procédé d'activation selon l'invention étant alors un traitement cellulaire extracorporel, pouvant notamment servir en laboratoire, par exemple pour l'étude des lymphocytes Tγ9δ2 ou de leurs propriétés, ou à des fins de diagnostic. L'invention s'étend aussi à une composition pour le diagnostic extracorporel (*ex vivo*) caractérisée en ce qu'elle comprend au moins un composé selon l'invention.

Ledit milieu peut être aussi intracorporel, l'activation des lymphocytes Tγ9δ2 ayant alors une utilité thérapeutique.

Plus particulièrement, ledit milieu est le sang périphérique d'un primate. L'invention s'étend donc en particulier à un procédé d'activation des lymphocytes Tγ9δ2 du sang périphérique d'un primate -notamment de l'homme- dans lequel on administre une quantité apte à activer les lymphocytes Tγ9δ2 d'au moins un composé selon l'invention. On administre donc au moins un composé selon l'invention par voie générale -notamment parentérale dans le sang périphérique-.

Ledit milieu peut aussi être un site cellulaire à traiter, et on administre au moins un composé selon l'invention directement au contact du site cellulaire à traiter (administration topique).

L'invention s'étend ainsi en particulier aux applications thérapeutiques des composés selon l'invention pour le traitement des pathologies des primates appartenant au groupe formé des cancers, des maladies infectieuses, notamment mycobactériennes (lèpre, tuberculose...) ; des parasitoses (paludisme...) ; des pathologies à syndrome d'immunodéficience (SIDA, ...). Selon l'invention, on administre une composition thérapeutique adaptée pour libérer dans le sang périphérique et/ou sur un site cellulaire à traiter une quantité d'au moins un composé selon l'invention apte à activer les lymphocytes Tγ9δ2. En effet, il a été démontré de façon générale dans l'art antérieur sus-cité qu'une composition ayant la propriété d'activer les lymphocytes Tγ9δ2 peut être avantageusement utilisée pour le traitement de ces pathologies.

De façon traditionnelle, dans tout le texte, les termes "thérapie" ou "thérapeutique" englobent non seulement les traitements curatifs ou les soins, mais également les traitements préventifs (prophylaxie) tels que la vaccination ainsi que le diagnostic intracorporel (administration à des fins de diagnostic). En effet, en permettant l'activation des lymphocytes Tγ9δ2, l'invention permet des traitements d'immunostimulation pouvant être avantageux aussi bien à titre prophylactique en empêchant le développement de cellules pathogènes sensibles aux lymphocytes Tγ9δ2, qu'à titre curatif en induisant la destruction de cellules pathogènes sensibles aux lymphocytes Tγ9δ2.

L'invention s'étend ainsi à une composition thérapeutique comprenant au moins un composé selon l'invention. Plus particulièrement, l'invention concerne une composition thérapeutique comprenant une quantité apte à être administrée à un primate -notamment au contact du sang périphérique ou par voie topique d'au moins un composé selon l'invention -notamment pour le traitement préventif ou curatif des pathologies sus-citées-. Une composition selon l'invention peut être une composition immunostimulante, ou un vaccin, les composés selon l'invention étant des antigènes activant les lymphocytes Tγ9δ2.

Avantageusement et selon l'invention, la composition thérapeutique est caractérisée en ce qu'elle comprend en outre une proportion d'interleukine -notamment d'interleukine 2- adaptée pour engendrer une croissance lymphocytaire dans le milieu où elle est destinée à être administrée.

Une composition thérapeutique selon l'invention peut être préparée sous une forme galénique apte à être administrée par voie générale, notamment par voie parentérale directement dans le sang périphérique d'un primate, avec au moins un composé selon l'invention en quantité adaptée pour activer les lymphocytes Tγ9δ2 et un ou plusieurs excipient(s) approprié(s). Compte tenu de la très faible valeur de la concentration active des composés selon l'invention (de l'ordre de 0,1 à 10 nM), une telle administration est envisageable sans risque de toxicité.

Une composition thérapeutique selon l'invention peut aussi être préparée sous une forme galénique appropriée pour son administration topique, directement au contact des cellules sensibles aux lymphocytes Tγ9δ2.

La forme galénique d'une composition thérapeutique selon l'invention est réalisée selon la voie d'administration choisie, par les techniques traditionnelles de formulation galénique. La quantité et la concentration de composé(s) selon l'invention, et la posologie sont déterminées par référence aux traitements chimiothérapeutiques connus des maladies à traiter, compte tenu de la bioactivité des composés selon l'invention vis-à-vis des lymphocytes Tγ9δ2, de l'individu à traiter, de la maladie concernée et des effets biologiques recherchés.

Avantageusement et selon l'invention, pour un composé bioactif à une concentration comprise entre 1nM et 10nM, on administre par voie générale une quantité de composé(s) selon l'invention comprise entre 0,1µg et 100µg -notamment entre 1µg et 10µg- par kilogramme de poids du patient.

Par ailleurs, il a été démontré *in vitro* que les composés selon l'invention ne présentent aucune toxicité générale même pour des concentrations pouvant aller jusqu'à 100µM, soit de l'ordre de 10⁵ fois la concentration bioactive. En outre, on sait que la catégorie biochimique de molécules à laquelle les composés selon l'invention appartiennent (phosphoesters) constitue une famille de composés métabolites rencontrés dans toute cellule vivante. Les composés selon l'invention ne présentent donc pas d'autres effets toxiques que ceux induits par leur bioactivité sur les lymphocytes Tγ9δ2.

En outre, certains composés selon l'invention présentent un poids moléculaire suffisamment faible (notamment inférieur à 500) pour être compatible avec leur élimination par voie rénale et urinaire.

Un exemple de formulation de composition thérapeutique injectable selon l'invention pour un primate de 1kg est le suivant : 5µg de 3-(iodométhyl)-3-butanol-1-yl-diphosphate (IHPP) dilués dans 0,5ml de tampon phosphate stérile à pH 7 amenés à 37°C.

On administre ainsi 5µg d'IHPP (composé de formule (2)) pour 1kg d'animal, correspondant à une concentration dans le sang circulant adaptée pour être supérieure à la concentration bioactive de l'IHPP (une concentration de 10nM d'IHPP correspondant à environ 5ng/ml).

Il est à noter que la majorité des excipients ou autres additifs pharmaceutiquement acceptables traditionnellement utilisés, sont chimiquement compatibles avec les composés selon l'invention.

Une composition thérapeutique selon l'invention peut aussi avantageusement comprendre un ou plusieurs autre(s) principe(s) actif(s), notamment pour procurer un effet synergique. En particulier, un composé selon l'invention peut faire office d'adjuvant de vaccin. La composition thérapeutique vaccinante selon l'invention est alors formée d'une composition vaccinante connue à laquelle on rajoute une quantité de composé(s) selon l'invention apte à activer les lymphocytes Tγ9δ2 qui non seulement pourront exercer directement leur activité anti-infectieuse, mais aussi activer les lymphocytes T effecteurs de la réponse vaccinale traditionnelle.

Une composition thérapeutique selon l'invention peut aussi incorporer elle-même des lymphocytes Tγ9δ2 de primates en culture dans un milieu compatible avec la croissance lymphocytaire T. Elle peut alors servir au traitement des primates, ou plus généralement des animaux vertébrés avec lesquels l'administration des lymphocytes Tγ9δ2 de primates peut être effectuée dans des conditions de compatibilité immunitaire vis-à-vis desdits lymphocytes Tγ9δ2 de primates. Une telle composition selon l'invention peut être administrée par voie générale, ou même par voie topique, au contact des cellules cibles pathogènes, sensibles auxdits lymphocytes Tγ9δ2 de primates.

L'invention s'étend aussi à l'utilisation d'au moins un composé selon l'invention pour la fabrication d'une composition thérapeutique selon l'invention, Plus particulièrement, l'invention porte sur l'utilisation d'au moins un composé selon l'invention pour la fabrication d'une composition thérapeutique destinée au traitement préventif ou curatif d'une pathologie de l'homme ou de l'animal vertébré produisant des cellules sensibles aux lymphocytes Tγ9δ2 des primates -notamment une pathologie sélectionnée dans le groupe formé des cancers, des maladies infectieuses, des parasitoses et des pathologies à syndrome d'immunodéficience-. A ce titre, l'invention s'étend aussi à l'utilisation d'au moins un composé selon l'invention pour la fabrication d'une composition thérapeutique destinée à être administrée -notamment au contact du sang périphérique ou par voie topique- à un primate -notamment à l'homme- pour le traitement préventif ou curatif d'une pathologie telle que mentionnée ci-dessus.

L'invention s'étend aussi à un procédé de fabrication d'une composition -notamment une composition thérapeutique- selon l'invention ayant la propriété d'activer les lymphocytes Tγ9δ2, dans lequel on utilise au moins un composé selon l'invention.

L'invention porte aussi sur un procédé de fabrication d'une composition thérapeutique destinée au traitement préventif ou curatif d'une pathologie de l'homme ou de l'animal vertébré produisant des cellules pathogènes sensibles aux lymphocytes Tγ9δ2 de primates, dans lequel on utilise au moins un composé selon l'invention. L'invention porte en particulier sur un procédé de fabrication d'une composition thérapeutique destinée à être administrée - notamment au contact du sang périphérique ou par voie topique- à un primate pour le traitement préventif ou curatif d'une pathologie produisant des cellules sensibles aux lymphocytes Tγ9δ2 -notamment une pathologie appartenant au groupe mentionné ci-dessus-, dans lequel on utilise au moins un composé selon l'invention.

Avantageusement et selon l'invention, dans un procédé de fabrication selon l'invention, on met au moins un composé selon l'invention au contact d'un milieu contenant des lymphocytes Tγ9δ2 de primates, et compatible avec la croissance lymphocytaire T, en une quantité adaptée pour activer ces lymphocytes Tγ9δ2 dans ce milieu. Avantageusement et selon l'invention, ledit milieu comprend une substance choisie parmi le sang des primates et les extraits de sang des primates. On obtient alors une composition thérapeutique contenant des lymphocytes Tγ9δ2 activés, permettant de réaliser une approche thérapeutique cellulaire.

Il est à noter que les composés selon l'invention sont halogénés et ne peuvent donc pas, pour cette seule raison, correspondre aux phosphoantigènes naturels, notamment aux molécules dites Tubag1, Tubag2, Tubag3 et Tubag4 obtenues comme décrit par WO 95/20673. Au demeurant, on démontre par exemple que ces phosphoantigènes naturels sont dégradés par l'eau de brome servant à la fabrication chimique des phosphobromohydrines selon l'invention. Les composés selon l'invention ne sont donc pas des antigènes naturels, mais sont des antigènes synthétiques activateurs des lymphocytes Tγ9δ2 à des concentrations du même ordre, et avec une efficacité semblable voire même supérieure à celle des antigènes naturels.

Il est aussi à noter que, contrairement à l'état de la technique tel qu'illustré par US-5639653 qui considérait que la présence d'un groupe alkyle ou alcène était indispensable pour activer les lymphocytes Tγ9δ2 humains, les inventeurs ont constaté qu'en détruisant la liaison alcène avec addition d'halogène, élément absent des composés biologiques naturels, une activation des lymphocytes Tγ9δ2 extrêmement forte et à très faible concentration est obtenue. En particulier, on constate que l'effet peut même dépasser celui des phosphoantigènes d'origine naturelle.

D'autres caractéristiques, buts et avantages de l'invention apparaissent à la lecture des exemples qui suivent donnés à titre non limitatifs uniquement à des fins de compréhension, ainsi que des figures dans lesquelles :
- la figure 1 est un graphe représentant des résultats obtenus dans l'exemple 10,
- la figure 2 est un graphe représentant des résultats obtenus dans l'exemple 11.

### EXEMPLE 1 : Fabrication du 3-(bromométhyl)-3-butanol-1-yl-diphosphate (BrHPP) :

### Préparation du 3-méthyl-3-butène-1-yl-tosylate (isopentènyl tosylate) :

Dans un réacteur en verre équipé pour la manipulation sous atmosphère inerte et soigneusement séché, sont introduits sous agitation magnétique (2,32 mmoles - 442mg) de chlorure de tosyle et (2,55 mmoles - 312 mg) de 4-(N,N-diméthylamino)pyridine dans 5 ml de dichlorométhane anhydre. A ce mélange, on ajoute lentement à l'aide d'une seringue et par l'intermédiaire d'un septum (2,32 mmoles - 200 mg) d'isopentènol en solution dans environ 1ml de dichlorométhane. La réaction est suivie par chromatographie sur couche mince de silice (gel de silice 60 F-254 - éluant : pentane/acétate d'éthyle 85/15 v/v - R_{f} (produit) = 0,4 et R_{f} (TsCI) = 0,5). Après environ 3 heures d'agitation sous atmosphère d'azote on dilue le mélange réactionnel dans un grand volume d'hexane (environ 100ml) ce qui entraîne la formation immédiate d'un précipité blanc. Le mélange est ensuite filtré et le filtrat concentré par évaporation sous pression réduite. La solution est diluée avec du diéthyl éther et filtrée à nouveau. Après évaporation du solvant, on obtient une huile jaunâtre. Le produit est purifié par chromatographie sur colonne préparatrice de silice (gel de silice 60 - éluant : pentane/acétate d'éthyle 85/15).(1,98 mmoles - 475 mg) de 3-méthyl-3-butène-1-yl -tosylate (85 % en rendement isolé) sont ainsi obtenus. Le composé (huile incolore) est stocké à + 4°C en milieu anhydre.

### Préparation du tris(tetra-n-butylammonium) hydrogènopyrophosphate :

(4,5 mmoles - 1g) de sel de disodium-dihydrogènopyrophosphate (Na₂H₂P₂O₇) sont dissous dans 10 ml d'eau déionisée froide préalablement ajustée à pH 9 par une solution d'ammoniaque 10mM. La solution est passée sur une colonne contenant (19 milliéquivalents - 4 g) de résine cationique DOWEX® 50-WX8 -200 (forme H⁺). La solution acide est éluée avec 15-20 ml d'eau déionisée froide à pH 9. La solution collectée est immédiatement titrée à pH 7,3 par une solution aqueuse d'hydroxyde de tétra-n-butylarnmonium (Bu₄NOH) à 40% . Après lyophilisation on obtient 4 g de sel de tétra-n-butylammonium sous la forme d'un solide blanc hygroscopique. Le sel est dissout dans 10 ml d'acétonitrile anhydre. La solution est ensuite filtrée puis séchée par évaporations successives du solvant sous pression réduite. On obtient ainsi une solution de tris(tetra-n-butylammonium) hydrogènopyrophosphate avec une pureté égale à 98 % (résultat déduit de l'analyse par chromatographie ionique - HPAEC). Le volume est ajusté afin d'obtenir une concentration en sel comprise entre 0,5 et 1M. La solution est stockée à -20 °C en milieu anhydre.

### Préparation du 3-méthyl-3-butène-1-yl-diphosphate (isopentènyl pyrophosphate):

Dans un réacteur en verre soigneusement séché, on introduit sous atmosphère d'azote 2,5 ml d'une solution de tris(tetra-n-butylammonium) hydrogènopyrophosphate à 0,7 M (1,75 mmoles) dans l'acétonitrile anhydre. Le réacteur est refroidi par un bain de glace puis on ajoute sous agitation magnétique et à l'aide d'une seringue (0,70 mmoles - 168 mg) de 3-méthyl-3-butène-1-yl-tosylate en solution dans un minimum d'acétonitrile (0,5 - 1M). Après introduction du tosylate, le bain de glace est retiré puis la réaction est laissée sous agitation à température ambiante. L'avancement de la réaction est alors suivi par chromatographie ionique (HPAEC). Après environ 3 heures, le solvant est évaporé sous pression réduite et le milieu réactionnel redissout dans 3 ml d'un mélange eau /2-propanol 98/2 (v/v). La solution est passée sur une colonne contenant (19 milliéquivalents - 4 g) de résine cationique DOWEX® 50-WX8 -200 (forme NH₄⁺) puis éluée avec 10 ml du mélange eau (pH 9)/2-propanol 98/2 (v/v). Après lyophilisation, on recueille un solide blanc contenant le produit brut.

### Purification :

Le pyrophosphate et les traces de monophosphate d'ammonium sont séparés du milieu par co-précipitation en présence d'hydrogénocarbonate d'ammonium. On dissout le produit brut obtenu à l'étape précédente dans 4 ml d'hydrogénocarbonate d'ammonium 0,1 M que l'on transfère dans un tube à centrifugation de 25 ml. On traite alors la solution avec 10 ml d'un mélange acétonitrile/2-propanol 1/1 (v/v) en agitant vigoureusement le mélange (vortex) pendant quelques minutes jusqu'à formation d'un précipité blanc. Le tube est ensuite centrifugé à 2000 tr/min à 10 °C pendant 5minutes. Le surnageant, dans lequel sont extraits les sels organiques, est conservé à +4°C. La procédure est renouvelée en redissolvant le précipité dans 3ml d'hydrogénocarbonate d'ammonium 0,1 M auxquels on ajoute 7 ml du mélange acétonitrile/2-propanol. Les deux surnageants sont regroupés et le solvant évaporé sous vide. On obtient un liquide huileux que l'on conserve à +4°C.

Le tosylate d'ammonium est séparé du milieu réactionnel par extraction avec le solvant chloroforme/méthanol 1/1 (v/v). Le liquide huileux de l'étape précédente est dissout dans 4 ml d'eau à pH 9 et traité avec lml de ce solvant par une procédure classique d'extraction répétée 3 fois. On élimine ensuite de la phase aqueuse les traces de solvant par évaporation sous pression réduite à 30 °C. On obtient sur la base de l'analyse par chromatographie ionique (HPAEC) un rendement de 83 % en 3-méthyl-3-butène-1-yl-diphosphate (0,58 mmoles - 172 mg). La solution est stockée à -20 °C.
Le produit est purifié ultérieurement selon les besoins par chromatographie d'échange d'anions sur cartouches Sep-Pak Accell Plus QMA (Waters®) de 360 mg à 10 grammes éluées successivement par des solutions aqueuses d'hydrogénocarbonate d'ammonium respectivement de 20 mM, 40 mM, 100 mM, puis 200 mM avec suivi chromatographique (HPAEC) des fractions éluées. Les fractions correspondant au produit purifié sont regroupées puis lyophilisées.

### Préparation du 3-(bromométhyl)-3-butanol-1-yl-diphosphate :

(0,34 mmoles - 100 mg) d'isopentènyl pyrophosphate (sel d'ammonium) en solution dans 2 ml d'eau déionisée de pH neutre sont traitées sous une hotte aspirante et à température ambiante par 1,9 ml (0,34 mmoles) de brome en solution aqueuse saturée (0,18 M). L'eau de brome est ajoutée progressivement et de préférence sur une solution froide du sel d'ammonium en agitant périodiquement jusqu'à décoloration de l'eau de brome. Dans le cas où le brome est ajouté en léger excès (coloration jaune persistante), la solution est transférée dans un ballon en verre puis placée quelques minutes sous pression réduite (évaporateur rotatif) à une température de 30 °C jusqu'à disparition de la coloration. Le produit 3-(bromométhyl)-3-butanol-1-yl-diphosphate est généré quantitativement (0,33 mmoles - 130 mg) ; ce résultat étant déduit de l'analyse par chromatographie ionique - HPAEC. Pour la mise en oeuvre de test biologiques, les solutions aqueuses ainsi obtenues sont filtrées puis neutralisées par passage sur colonne de résine cationique. Les ions bromures peuvent être éliminés de la solution en utilisant un dispositif DIONEX® composé d'une cartouche OnGuard®-Ag fixée sur une cartouche OnGuard®-H. Ce dispositif permet de retenir sélectivement les ions halogénures de la solution. Pour la mise en oeuvre de tests biologiques, les solutions aqueuses du produit sont stérilisées par filtration sur filtre de 0,2 µm et stockées (de préférence avec un pH neutre à légèrement acide) à -20 °C. Dans le cas de tests réalisés *in vivo*, les solutions sont préalablement passées sur une colonne de résine cationique DOWEX® 50-WX8-200 (forme Na⁺) éluée par deux volumes de colonne d'eau déionisée.

### EXEMPLE 2 : Fabrication du 3-(iodométhyl)-3-butanol-1-yl-diphosphate (IHPP) :

### Préparation de l'eau iodée :

Une solution d'eau iodée de l'ordre de 0,5 à 1 mM est préparée par sonication prolongée (15 minutes environ) de quelques cristaux d'iode dans une solution d'eau déionisée et filtration. Pour des essais portant sur de plus grandes quantités, des solutions plus concentrées en iode peuvent être obtenues en rajoutant une faible proportion d'alcool à la solution aqueuse initiale. L'eau iodée est ensuite titrée par le thiosulfate de sodium avec de l'empois d'amidon comme indicateur coloré.

### Préparation du 3-(iodométhyl)-3-butanol-1-yl-diphosphate :

Une micromole (1 ml d'une solution millimolaire) d'isopentènyl pyrophosphate préparé selon l'exemple 1 sous forme de sel d'ammonium en milieu aqueux ou hydroalcoolique de pH neutre est traitée à température ambiante par ajout d'une micromole d'iode en solution aqueuse (1,43 ml d'eau iodée à 0,7 mM). La solution est placée 30 minutes à température ambiante, puis 30 minutes à +4 °C en effectuant périodiquement une agitation vigoureuse. Après décoloration de l'eau iodée, le produit 3-(iodométhyl)-3-butanol-1-yl-diphosphate est généré quantitativement (1 micromole dans environ 2,5 ml). La solution est ensuite traitée comme dans l'exemple 1 pour la mise en oeuvre de tests biologiques et/ou pour la réalisation d'essais *in vivo* et stockée à - 20°C.

### EXEMPLE 3 : Fabrication du 3-(chlorométhyl)-3-butanol-1-yl-diphosphate (C1HPP) :

### Préparation de l'eau de chlore :

La solution d'eau de chlore est préparée par barbotage de chlore gazeux dans une solution d'eau déionisée. Elle est ensuite titrée par le thiosulfate de sodium en présence d'iodure de potassium en excès et avec de l'empois d'amidon comme indicateur coloré.

### Préparation du 3-(chlorométhyl)-3-butanol-1-yl-diphosphate :

Une micromole (1 ml d'une solution millimolaire) d'isopentènyl pyrophosphate préparé selon l'exemple 1 sous forme de sel d'ammonium en milieu aqueux ou hydroalcoolique de pH neutre est traitée à température ambiante par ajout d'une micromole de chlore en solution aqueuse (72 µl d'eau de chlore à 14 mM). Après 30 minutes à température ambiante avec agitation périodique, le produit 3-(chlorométhyl)-3-butanol-1-yl-diphosphate est généré quantitativement (ici 1 micromole dans environ 1,1 ml). La solution est ensuite traitée comme dans l'exemple 1 pour la mise en oeuvre de tests biologiques et/ou pour la réalisation d'essais *in vivo* et stockée à -20 °C.

### EXEMPLE 4 : Fabrication du 3-(bromométhyl)-3-butanol-1-yl-triphosphate (BrHPPP) :

### Préparation du tetrakis(tetra-n-butylammonium) hydrogènotriphosphate :

(2,1 mmoles - 1g) de sel de pentasodium tripolyphosphate hexahydrate (Na₅P₃O₁₀.6H₂O) sont dissous dans 10 ml d'eau déionisée froide préalablement ajustée à pH 9 par une solution d'ammoniaque 10mM. La solution est passée sur une colonne contenant (21 milliéquivalents - 4,4 g) de résine cationique DOWEX® 50-WX8 (forme H⁺). La solution acide est éluée avec 20-25 ml d'eau déionisée froide à pH 9. La solution collectée est immédiatement titrée à pH 7,0 par une solution aqueuse d'hydroxyde de tétra-n-butylammonium (Bu₄NOH) à 40% . Après lyophilisation on obtient 2,5 g de sel de tétra-n-butylammonium sous la forme d'un solide blanc hygroscopique. Le sel est dissout dans 10 ml d'acétonitrile anhydre. La solution est ensuite filtrée puis séchée par évaporations successives du solvant sous pression réduite. On obtient ainsi une solution de tetrakis(tetra-n-butylammonium) hydrogènotriphosphate avec une pureté égale à 95 % (résultat déduit de l'analyse par chromatographie ionique - HPAEC). Le volume est ajusté afin d'obtenir une concentration en sel comprise entre 0,5 et 1M. La solution est stockée à -20 °C en milieu anhydre.

### Préparation du 3-méthyl-3-butène-1-yl-triphosphate (isopentènyl triphosphate) :

En suivant la procédure décrite pour la préparation du 3-méthyl-3-butène-1-yl-diphosphate (exemple 1), on fait réagir sous atmosphère d'azote 2 mmoles d'une solution molaire de tetrakis(tetra-n-butylammonium) hydrogènotriphosphate avec (1 mmole - 240 mg) de 3-méthyl-3-butène-1-yl-tosylate préparé selon l'exemple 1 dans 4 ml d'acétonitrile anhydre pendant 24 heures. En utilisant une procédure de purification par précipitation-extraction analogue à celle appliquée au 3-méthyl-3-butène-1-yl-diphosphate, on obtient sur la base de l'analyse par chromatographie ionique (HPAEC) un rendement de 74% en 3-méthyl-3-butène-1-yl-triphosphate (0,74 mmoles - 292 mg). Pour la préparation de composés phosphohalohydrines selon l'invention dans le cadre de tests biologiques, on utilise une fraction du produit obtenu à ce stade que l'on purifie par HPAEC sur colonne IonPac® AS11 en cumulant plusieurs passages chromatographiques. On prépare de cette manière environ 2 ml d'une solution aqueuse millimolaire de pH neutre de 3-méthyl-3-butène-1-yl-triphosphate sous forme de sel d'ammonium.

### Préparation du 3-(bromométhyl)-3-butanol-1-yl-triphosphate :

300 nmoles (300 µl d'une solution millimolaire) d'isopentènyl triphosphate sont traitées à température ambiante par ajout de 300 nmoles de brome en solution aqueuse saturée (1,7 µl d'eau de brome à 180 mM). On observe une décoloration quasi instantanée de l'eau de brome. Après agitation du mélange et décoloration de l'eau de brome (quasi instantanée), le produit 3-(bromométhyl)-3-butanol-1-yl-triphosphate est généré quantitativement (300 µl d'une solution millimolaire). La solution est ensuite traitée comme dans l'exemple 1 pour la mise en oeuvre de tests biologiques et/ou pour la réalisation d'essais *in vivo* et stockée à -20 °C.

### EXEMPLE 5 : Fabrication du 3-(iodométhyl)-3-butanol-1-yl-triphosphate (IHPPP) :

300 nmoles (300 µl d'une solution millimolaire) d'isopentènyl triphosphate préparé selon l'exemple 4 sont traitées en milieu aqueux ou hydroalcoolique de pH neutre par ajout de 429 µl d'eau iodée à 0,7 mM préparée selon l'exemple 2. La solution est laissée 30 minutes à température ambiante en effectuant périodiquement une agitation vigoureuse. Après décoloration de l'eau iodée, le produit 3-(iodométhyl)-3-butanol-1-yl-triphosphate est généré quantitativement (729 µl d'une solution à 411 µM). La solution est ensuite traitée comme dans l'exemple 1 pour la mise en oeuvre de tests biologiques et/ou pour la réalisation d'essais *in vivo* et stockée à -20 °C.

### EXEMPLE 6 : Fabrication du α,γ di-[3-(bromométhyl)-3-butanol-1-yl]-triphosphate (diBrHTP) :

### Préparation du α,γ di-[3-méthyl-3-butène-1-yl]-triphosphate :

En suivant la procédure décrite pour la préparation du 3-méthyl-3-butène-1-yl -diphosphate (exemple 1), on fait réagir sous atmosphère d'azote 0,5 mmoles d'une solution molaire de tetrakis(tetra-n-butylammonium) hydrogènotriphosphate (préparé selon l'exemple 4) avec (1 mmole - 240 mg) de 3-méthyl-3-butène-1-yl-tosylate (préparé selon l'exemple 1) dans 4 ml d'acétonitrile anhydre pendant 24 heures. En utilisant une procédure de purification par précipitation-extraction analogue à celle appliquée au 3-méthyl-3-butène-1-yl-diphosphate, on obtient sur la base de l'analyse par chromatographie ionique (HPAEC) un rendement de 81 % en α,γ di-[3-méthyl-3-butène-1-yl]-triphosphate (0,4 mmoles - 178 mg). Pour la préparation de composés phosphohalohydrines selon l'invention dans le cadre de tests biologiques, on utilise une fraction du produit obtenu à ce stade que l'on purifie par HPAEC sur colonne IonPac® AS11 en cumulant plusieurs passages chromatographiques. Avant chaque passage chromatographique et pour une meilleure isolation du produit, on effectue un traitement enzymatique à la phosphatase alcaline de la fraction à purifier pour dégrader l'isopentènyl triphosphate, qui est un sous produit de la réaction. On prépare de cette manière environ 1 ml d'une solution aqueuse millimolaire de pH neutre de α,γ di-[3-méthyl-3-butène-1-yl]-triphosphate sous forme de sel d'ammonium.

### Préparation du α,γ di-[3-(bromométhyl)-3-butanol-1-yl]-triphosphate :

250 nmoles (250 µl d'une solution millimolaire) de α,γ di-[3-méthyl-3-butène-1-yl]-triphosphate sont traitées à température ambiante par ajout de 250 nmoles de brome en solution aqueuse saturée (1,4 µl d'eau de brome à 180 mM). Après agitation du mélange et décoloration de l'eau de brome (quasi instantanée), le produit α,γ di-[3-(bromométhyl)-3-butanol-1-yl]-triphosphate est généré quantitativement (environ 250 µl d'une solution millimolaire). La solution est ensuite traitée comme dans l'exemple 1 pour la mise en oeuvre de tests biologiques et/ou pour la réalisation d'essais *in vivo* et stockée à -20 C.

### EXEMPLE 7 : Fabrication du α,γ di-[3-(iodométhyl)-3-butanol-1-yl]-triphosphate (diIHTP) :

250 nmoles (250 µl d'une solution millimolaire) de α,γ di-[3-méthyl-3-butène-1-yl]-triphosphate préparé selon l'exemple 6 sont traitées en milieu aqueux ou hydroalcoolique de pH neutre par ajout de 358 µl d'eau iodée à 0,7 mM préparée selon l'exemple 2. La solution est laissée 30 minutes à température ambiante en effectuant périodiquement une agitation vigoureuse. Après décoloration de l'eau iodée, le produit α,γ di-[3-(iodométhyl)-3-butanol-1-yl]-triphosphate est généré quantitativement (608 µl d'une solution à 411 µM). La solution est ensuite traitée comme dans l'exemple 1 pour la mise en oeuvre de tests biologiques et/ou pour la réalisation d'essais *in vivo* et stockée à -20 °C.

### EXEMPLE 8 : Fabrication de l'uridine 5'-triphosphate γ-[3-(iodométhyl)-3-butanol-1-yl] :

### Préparation de l'uridine 5'-triphosphate y-[3-méthyl-3-butène-1-yl] :

Ce produit est préparé selon la procédure décrite par KNORRE D. C. *et al.* "General Method for the synthesis of ATP Gamma-derivatives" Febs Letters, 1976, 70-1, 105-108, à partir de 40 µmoles d'uridine-5'-triphosphate (UTP) (sel de triéthylammonium) en présence d'un excès d'isopentènol. Pour la préparation de composés phosphohalohydrines selon l'invention dans le cadre de tests biologiques, une fraction du produit obtenu est purifié par HPAEC sur colonne IonPac® AS11 en cumulant plusieurs passages chromatographiques. Avant chaque passage chromatographique et pour une meilleure isolation du produit, on effectue un traitement enzymatique à la phosphatase alcaline de la fraction à purifier pour dégrader les sous produits (UDP et UMP) et l'UTP n'ayant pas réagi. On prépare de cette manière environ 500µl d'une solution aqueuse à 300 µM de pH neutre de l'uridine 5'-triphosphate γ-[3-méthyl-3-butène-1-yl] sous forme de sel d'ammonium.

### Préparation de l'uridine 5'-triphosphate γ-[3-(iodométhyl)-3-butanol-1-yl]:

75 nmoles (250 µl d'une solution 300 µM) d'uridine 5'-triphosphate γ-[3-méthyl-3-butène-1-yl] sous forme de sel d'ammonium sont traitées en milieu aqueux de pH neutre par ajout de 108 µl d'eau iodée à 0,7 mM préparée selon l'exemple 2. La solution est laissée 20 minutes à température ambiante en effectuant périodiquement une agitation vigoureuse. Après décoloration de l'eau iodée, le produit de l'uridine 5'-triphosphate y-[3-(iodométhyl)-3-butanol-1-yl] est généré quantitativement (environ 360 µl d'une solution à 200 µM). La solution est ensuite traitée comme dans l'exemple 1 pour la mise en oeuvre de tests biologiques et/ou pour la réalisation d'essais *in vivo* et stockée à -20 °C.

### EXEMPLE 9: Fabrication du α,β di-[3-bromométhyl-3-butanol-1-yl]-diphosphate :

Ce produit est préparé selon une procédure analogue à celle décrite dans l'exemple 6, le tetrakis(tétra-n-butylammonium) hydrogénotriphosphate étant remplacé par le tris(tétra-n-butylammonium) hydrogénopyrophosphate (préparé selon l'exemple 1) pour la préparation du composé intermédiaire : α,β di-[3-méthyl-3-butène-1-yl]-diphosphate.

### EXEMPLE 10 : Mesure de l'activité antigénique par stimulation de la prolifération des lymphocytes Tγ9δ2 en culture :

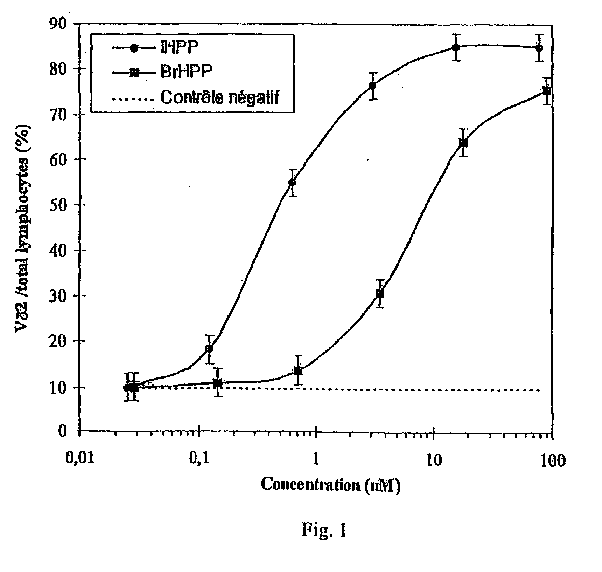

Dans une culture in vitro de 10⁶ lymphocytes T totaux dans 1ml, séparés à partir du sang d'un donneur humain sain adulte et contenant initialement de 1-5% de lymphocytes Tγ9δ2 en milieu de culture adéquat (RPMI 1640+10% de sérum humain inactivé et 50 U/ml d'interleukine 2 humaine (hIL-2), on rajoute 20 microlitres de solution aqueuse du composé selon l'invention amené à la concentration finale spécifiée dans l'essai. Après 4 jours de culture, on rajoute par millilitre de milieu de culture 50 U d'hIL-2. Après huit jours les cellules sont énumérées, collectées, lavées par un tampon phosphate, et les cellules de type Tγ9δ2 sont révélées dans la culture par un marquage avec les réactifs commerciaux usuels (Anticorps monoclonaux fluorescéinés) et leur proportion déterminée par une analyse de cytométrie en flux. On prend en compte soit le changement de la proportion, soit l'augmentation numérique des cellules Tγ9δ2 dans des cultures en présence du composé selon l'invention par rapport à des cultures exemptes de composé selon l'invention. On représente le résultat de ces essais en traçant les courbes de ces valeurs (ordonnées figure 1) en fonction de la concentration en échelle logarithmique de composé selon l'invention mis en culture (abscisses figure 1).

La figure 1 représente les résultats obtenus avec les composés selon l'invention obtenus aux exemples 1 (BrHPP) et 2 (IHPP), la ligne en pointillés représentant un contrôle négatif (valeur obtenue en l'absence de composé selon l'invention).

Le tableau I suivant illustre les valeurs de DE50, dose efficace à 50 % de l'effet maximal d'amplification lymphocytaire polyclonale obtenu comme indiqué ci-dessus, avec différents composés selon l'invention.

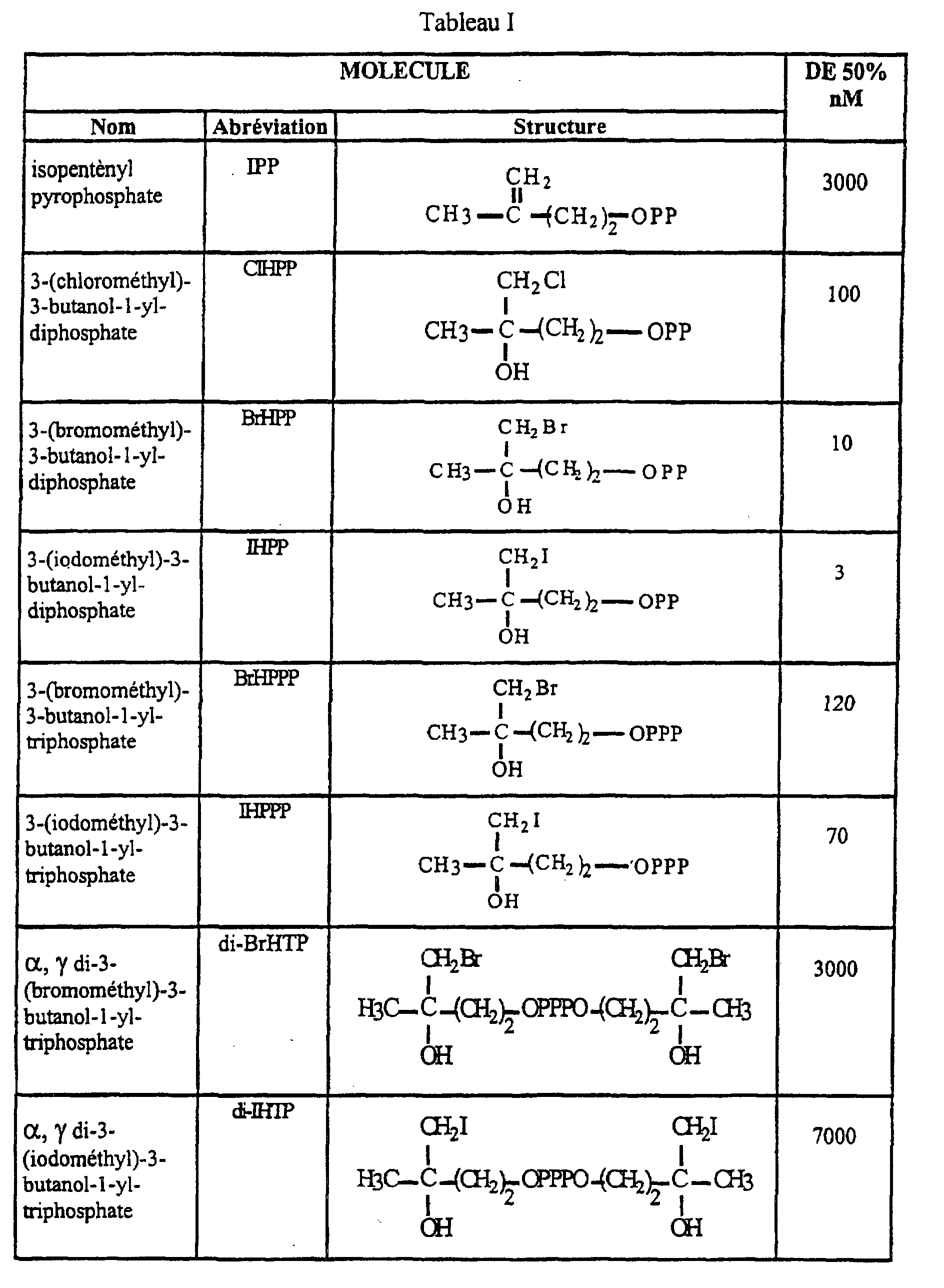

### EXEMPLE 11 : Mesure de l'activité antigénique par stimulation de la cytotoxicité induite :

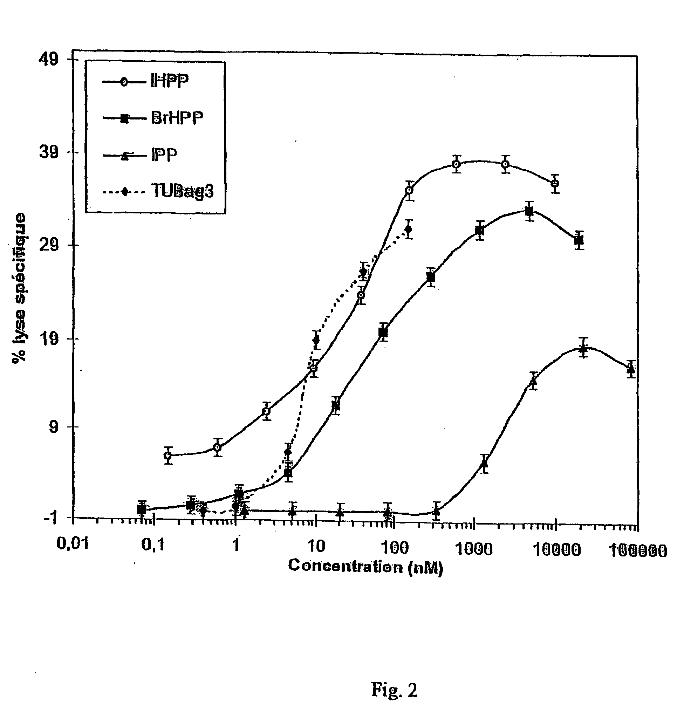

On compare l'activité cytotoxique spécifique d'un clone de lymphocyte Tγ9δ2 mesurée selon le test de cytotoxicité induite, cette activité étant simulée avec des concentrations décroissantes du phosphoantigène Tubag3 obtenu comme décrit par WO 95/20673 (courbe représentée par des losanges noirs figure 2), du composé BrHPP selon l'invention obtenu à l'exemple 1 (courbe représentée par des carrés noirs figure 2), du composé IHPP selon l'invention obtenu à l'exemple 2 (courbe représentée par des ronds blancs figure 2), et de l'isopentènyl pyrophosphate IPP (courbe représentée par des triangles noirs figure 2).

On constate que les composés selon l'invention sont actifs à une concentration de l'ordre de 10 nM du même ordre que celle du phosphoantigène naturel Tubag3, alors que l'IPP de l'art antérieur est actif à une concentration de l'ordre de 3 µM, soit 300 fois plus élevée.

### EXEMPLE 12 : Toxicité de BrHPP (sel de sodium):

Huit souris de 30g ont reçu une injection intraveineuse (veine caudale) de 300 µl de tampon PBS contenant 1 mg de BrHPP (sel de sodium). On n'observe aucun signe de choc ou de pyrogénicité : 8 souris sont survivantes à 30 jours; aucune variation significative du poids des souris n'est notée au cours de l'étude. La toxicité est donc inférieure à 12,5 % pour une dose de 33,4 g de BrHPP (Na⁺) par kilogramme d'animal.

### EXEMPLE 13 : Pyrogénicité de BrHPP (sel de sodium):

Cet essai est realisé selon un protocole défini par les normes ISO-10993-11 (1993) et USP XXIII. Il s'agit de l'évaluation de la réaction fébrile chez le lapin, après administration intraveineuse du BrHPP (sel de sodium) à 400 µg/ml (soit 10 ml d'extrait par kilogramme) à trois lapins. La température est mesurée toutes les 30 minutes durant 3 heures débutant 30 minutes après l'injection.
Un seul lapin a présenté une augmentation de température supérieure à 0.5°C (seuil minimum). A l'injection de la solution à 400 µg/ml, les lapins ont présenté un effet secondaire se traduisant par des tremblements musculaires. Un autre lapin injecté avec la même solution diluée 1:40 soit donc à 10 µg/ml en 19 minutes n'a présenté aucune réaction à l'injection.

### EXEMPLE 14 : Bioactivité de BrHPP (sel de sodium) in vivo chez le macaque :

Ces essais sont réalisés sur des singes *Macaca fascicularis* (males de 3 à 4 kg) sous anesthésie par Zoletil (zolazepam)® 15 mg/kg -Atropine 0.01mg/kg. Les lots de BrHPP (forme Na⁺) sont préalablement contrôlés en pureté (>99%), quantifiés et ont été évalués par les tests de pyrogénicité de l'exemple 12. Les animaux sont injectés par intraveineuse rapide (2 à 5 minutes dans la veine saphène) avec 0.1 mg/kg de BrHPP (soit 0,3-0,4 mg par animal) et dilués dans 10 ml de Ringer-Lactate. On injecte également 90.000 unités d'interleukine 2 (IL2) par animal en sous-cutané. On procède à ces injections une fois par jour pendant quatre jours. Des animaux de contrôle ne reçoivent que l'injection sous-cutanée d'IL2 une fois par jour durant quatre jours. On compare entre les divers animaux la proportion de lymphocytes Tγ9δ2 parmi les lymphocytes totaux, dans les trois situations suivantes :
- dans le sang de l'animal,
- dans une culture de lymphocytes totaux prélevés de l'animal avec BrHPP seul,
- dans une culture de lymphocytes totaux prélevés de l'animal avec BrHPP +IL2.
Les résultats sont donnés dans le tableau II suivant :

**Tableau II**

| animaux injectés: | % de lymphocytes Tγ9δ2 sanguins *in vivo* ( 7 jours après injection) | réactivité *ex vivo* des lymphocytes Tγ9δ2 (% après 10j de culture in vitro) | |
|---|---|---|---|
| | | amplification par 40 nM BrHPP | amplification par 40 nM BrHPP+IL2 |
| Naifs ou IL2 seule | ∼1%* [0.8-1.4] | 1% | ∼ 8% [4.7-12] |
| BrHPP seul | ∼1% | 1% | ∼11% [10-12] |
| 2 fois BrHPP seul (*1 injection + 1 rappel)* | ∼1% | 1% | 40.2% |
| BrHPP + IL2 | 7 % [5-10] | 10% | 50.0 % |

On constate que l'injection intraveineuse unique ou répétée de BrHPP seul préactive les lymphocytes Tγ9δ2 du singe mais ne stimule pas leur croissance chez l'animal. L'injection du BrHPP seul provoque l'état de préactivation des lymphocytes Tγ9δ2, qui se manifeste notamment par l'expression des récepteurs du facteur de croissance IL2 à leur surface cellulaire. Par contre, l'injection intraveineuse de BrHPP en présence d'IL2 sous-cutanée préactive ces mêmes cellules et provoque leur croissance *in vivo.* L'injection du facteur de croissance IL2 seul ne provoque pas de réponse spécifique des lymphocytes Tγ9δ2.

## Revendications

1. Composés comprenant au moins un groupement phosphohalohydrine de formule : 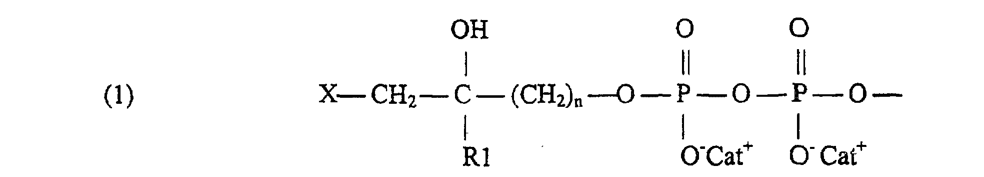 où
X est un halogène choisi parmi I, Br, Cl,
R1 est choisi parmi ―CH₃ et ―CH₂―CH₃,
Cat+ est un cation organique ou minéral,
et n est un nombre entier compris entre 2 et 20.

2. Composés phosphohalohydrines selon la revendication 1, de formule :

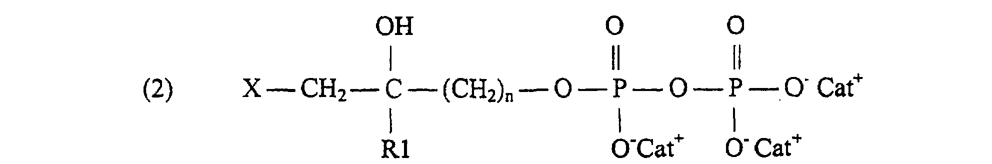

3. Composés phosphohalohydrines selon la revendication 1, de formule :

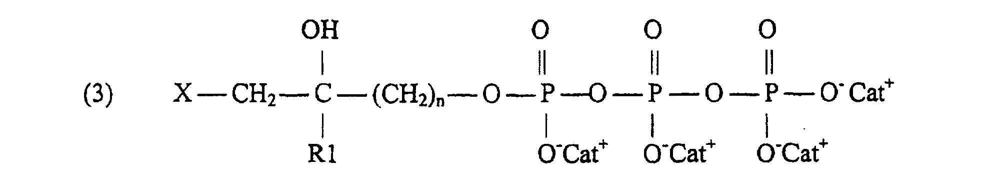

4. Composés phosphohalohydrines selon la revendication 1, de formule : 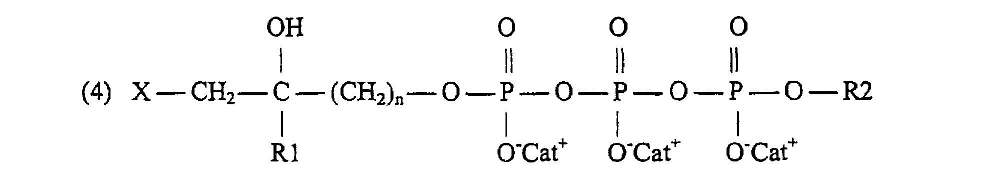 où R2 est un substituant organique ou minéral choisi dans le groupe formé des substituants qui n'empêchent pas la formation de la fonction halohydrine 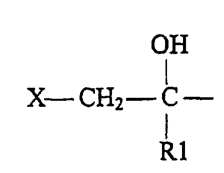 à partir de la fonction alcène 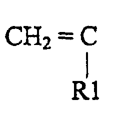 et de l'halogèneX₂ en présence d'eau ; et des substituants pour lesquels il existe un composé R2-O-Y non réactif sur la fonction halohydrine du composé de formule : 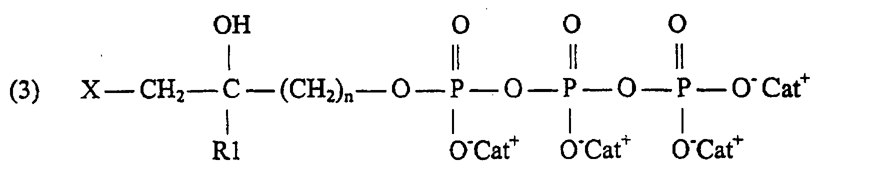 et choisi pour que R2-O-Y puisse réagir sur le phosphate terminal de ce composé (3) pour obtenir le composé (4).

5. Composés selon l'une des revendications 1 et 4, comprenant en outre au moins un groupement choisi dans le groupe formé des dérivés des nucléosides, des oligonucléotides, des acides nucléiques (ARN, ADN), des acides aminés, des peptides, des protéines, des monosaccharides, des oligosaccharides, des polysaccharides, des acides gras, des lipides simples, des lipides complexes, de l'acide folique, de l'acide tétrahydrofolique, des acides phosphoriques, de l'inositol, des vitamines, des co-enzymes, des flavonoïdes, des aldéhydes, des époxydes et des halohydrines.

6. Composés selon les revendications 4 et 5, dans lesquels R2 est choisi dans le groupe formé des dérivés des nucléosides, des oligonucléotides, des acides nucléiques (ARN, ADN), des acides aminés, des peptides, des protéines, des monosaccharides, des oligosaccharides, des polysaccharides, des acides gras, des lipides simples, des lipides complexes, de l'acide folique, de l'acide tétrahydrofolique, des acides phosphoriques, de l'inositol, des vitamines, des co-enzymes, des flavonoïdes, des phosphohalohydrines selon la formule (1), des aldéhydes, des époxydes et des halohydrines.

7. Composés phosphohalohydrines selon la revendication 1, de formule: 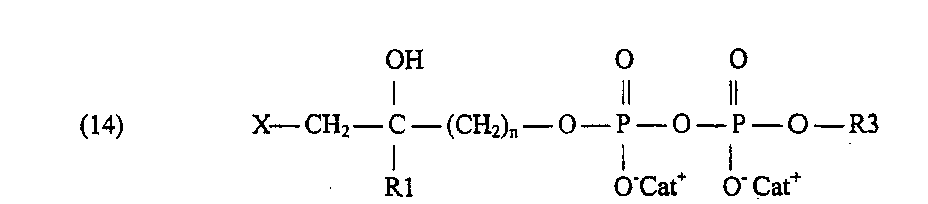 où
X est un halogène choisi parmi l'iode, le brome et le chlore,
RI est choisi parmi ―CH₃ et ―CH₂―CH₃,
Cat⁺ représente un (ou des) cation(s) organique(s) ou minéral(aux) (y compris le proton) identiques ou différents dans le même composé,
n est un nombre entier compris entre 2 et 20,
R3- est choisi parmi :
- un groupement halohydrine de formule : 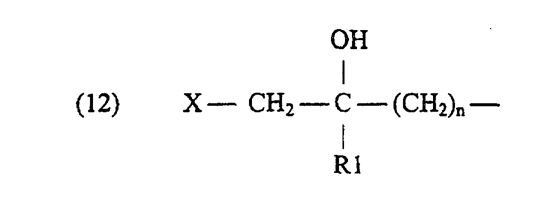
- un groupement époxyde de formule : 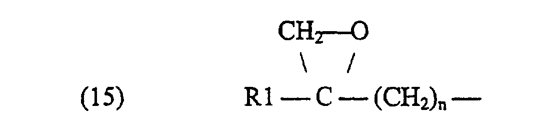
- un groupement alcène de formule : 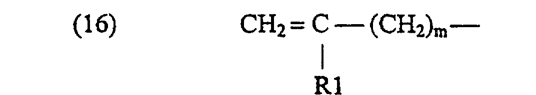
m étant un nombre entier compris entre 1 et 20.

8. Composés selon l'une des revendications 1 à 7 pour leur utilisation comme agent activateur des lymphocytes Tγ9δ2.

9. Composés selon l'une des revendications 1 à 8 pour leur utilisation à titre de substances thérapeutiquement actives.

10. Composés selon l'une des revendications 1 à 9 pour leur utilisation dans une composition thérapeutique immunostimulante ou un vaccin pour les primates.

11. Composés selon l'une des revendications 1 à 10 pour leur utilisation à titre d'antigènes des lymphocytes Tγ9δ2 dans une composition thérapeutique -notamment une composition immunostimulante ou un vaccin- pour les primates.

12. Procédé de fabrication d'un composé comprenant au moins un groupement phosphohalohydrine de formule : 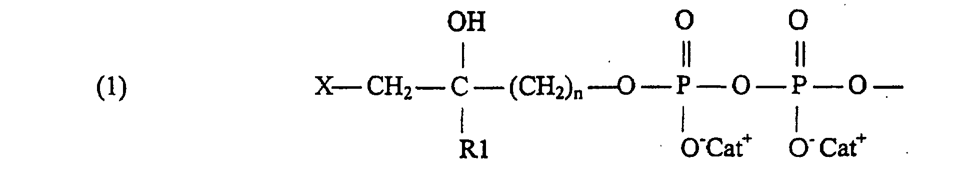 où
X est un halogène choisi parmi I, Br, Cl,
R1 est choisi parmi ―CH₃ et ―CH₂―CH₃,
Cat⁺ est un cation organique ou minéral,
et n est un nombre entier compris entre 2 et 20,
**caractérisé en ce qu'**on fait réagir l'halogène X₂ en présence d'eau avec un composé de départ comprenant au moins un groupement alcène phosphaté de formule :

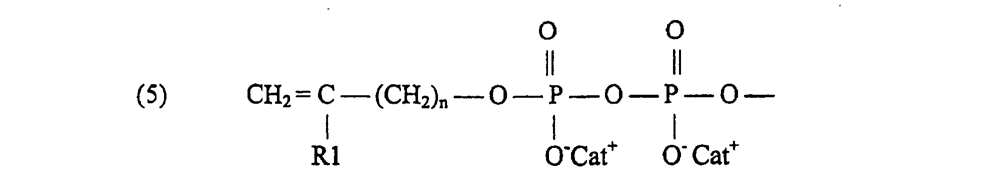

13. Procédé selon la revendication 12, **caractérisé en ce qu'**on fait réagir en milieu aqueux ou hydroalcoolique, à pH neutre, à une température inférieure à 30°C, une solution aqueuse de l'halogène X₂ avec un sel formé du composé de départ.

14. Procédé selon la revendication 13, **caractérisé en ce qu'**on effectue la réaction sous pression atmosphérique à une température comprise entre 0°C et 25°C.

15. Procédé selon l'une des revendications 12 à 14, **caractérisé en ce que** le composé de départ est un sel de formule :

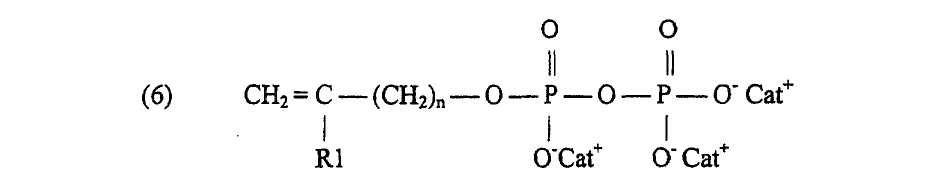

16. Procédé selon l'une des revendications 12 à 14, **caractérisé en ce que** le composé de départ est un sel de formule :

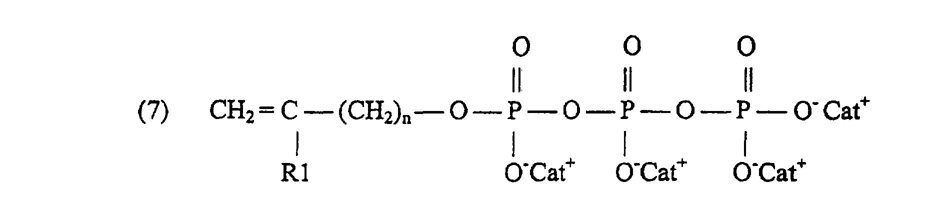

17. Procédé selon la revendication 16, **caractérisé en ce que** dans une étape ultérieure, on fait réagir le composé obtenu : 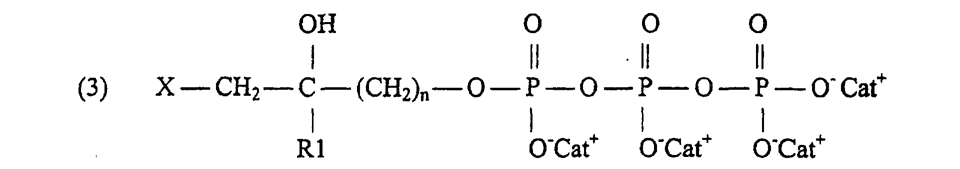 avec un composé R2-O-Y, où -O-Y est un groupement partant, et R2 est un substituant organique ou minéral choisi pour que R2-O-Y puisse former, par réaction sur le composé (3), le composé de formule :

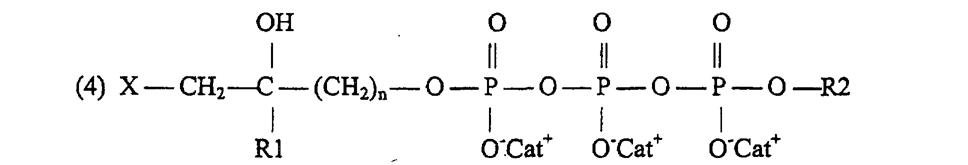

18. Procédé selon l'une des revendications 12 à 14, **caractérisé en ce que** le composé de départ est un sel de formule : 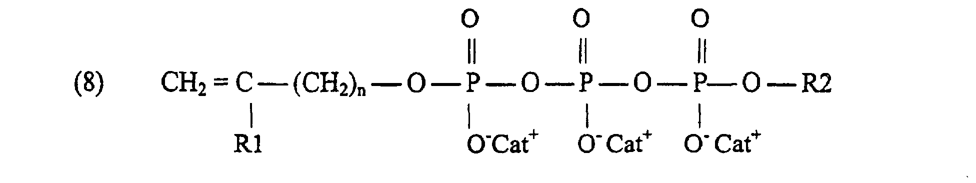 où R2 est un substituant organique ou minéral adapté pour ne pas empêcher la formation de la fonction halohydrine 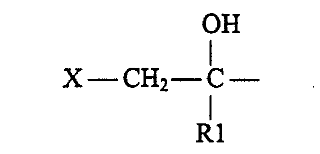 à partir de la fonction alcène 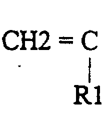 et de l'halogène X₂ en présence d'eau.

19. Composition pour le diagnostic extracorporel, caractérisée en qu'elle comporte au moins un composé selon l'une des revendications 1 à 7.

20. Composition thérapeutique, **caractérisée en ce qu'**elle comporte au moins un composé selon l'une des revendications 1 à 7.

21. Composition thérapeutique, **caractérisée en ce qu'**elle comprend une quantité apte à être administrée à un primate -notamment au contact du sang périphérique ou par voie topique- d'au moins un composé selon l'une des revendications 1 à 7.

22. Composition selon l'une des revendications 19 à 21, **caractérisée en ce qu'**elle comprend en outre des lymphocytes Tγ9δ2 de primates.

23. Composition selon l'une des revendications 19 à 22, **caractérisée en ce qu'**elle comprend en outre une proportion d'interleukine adaptée pour engendrer une croissance lymphocytaire dans le milieu où elle est destinée à être administrée.

24. Procédé de fabrication d'une composition ayant la propriété d'activer les lymphocytes Tγ9δ2, dans lequel on utilise au moins un composé selon l'une des revendications 1 à 7.

25. Procédé de fabrication d'une composition thérapeutique destinée au traitement préventif ou curatif d'une pathologie produisant des cellules pathogènes sensibles aux lymphocytes Tγ9δ2, dans lequel on utilise au moins un composé selon l'une des revendications 1 à 7.

26. Procédé de fabrication d'une composition thérapeutique destinée à être administrée à un primate pour le traitement préventif ou curatif d'une pathologie produisant des cellules sensibles aux lymphocytes Tγ9δ2, dans lequel on utilise au moins un composé selon l'une des revendications 1 à 7.

27. Procédé de fabrication d'une composition thérapeutique destinée à être administrée à un primate pour le traitement préventif ou curatif d'une pathologie sélectionnée dans le groupe formé des cancers, des maladies infectieuses, des parasitoses, et des pathologies à syndrome d'immunodéficience, dans lequel on utilise au moins un composé selon l'une des revendications 1 à 7.

28. Procédé selon l'une des revendications 24 à 27, dans lequel on met au moins un composé selon l'une des revendications 1 à 7 au contact d'un milieu contenant des lymphocytes Tγ9δ2, et compatible avec la croissance lymphocytaire T, en une quantité adaptée pour activer ces lymphocytes Tγ9δ2 dans ce milieu.

29. Procédé selon la revendication 28, dans lequel ledit milieu comprend une substance choisie parmi le sang des primates et les extraits de sang des primates.

30. Procédé d'activation extracorporelle des lymphocytes Tγ9δ2 de primates, dans lequel on met les lymphocytes Tγ9δ2 au contact d'au moins un composé selon l'une des revendications 1 à 7, dans un milieu extracorporel contenant les lymphocytes Tγ9δ2 compatible avec la croissance lymphocytaire T.

31. Procédé selon la revendications 30, dans lequel on utilise au moins un composé selon l'une des revendications 1 à 7 à une concentration dans le milieu qui procure une activation de la prolifération polyclonale des lymphocytes Tγ9δ2.

32. Procédé selon l'une des revendications 30 à 31, dans lequel on introduit dans le milieu une proportion d'interleukine adaptée pour engendrer une croissance lymphocytaire dans le milieu.

## Patentansprüche

1. Verbindungen, umfassend wenigstens eine Phosphohalohydringruppe der Formel: 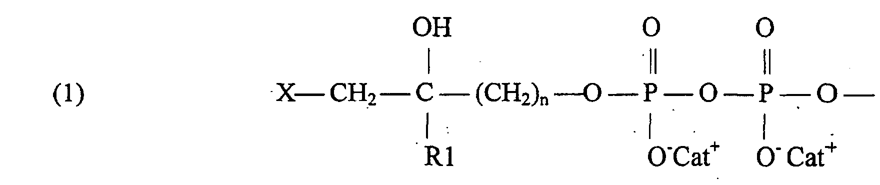 wobei
X ein Halogen ist, ausgewählt aus I, Br, Cl,
R1 aus -CH₃ und -CH₂-CH₃ ausgewählt ist,
Cat+ ein organisches oder mineralisches Kation ist,
und n eine ganze Zahl zwischen 2 und 20 ist.

2. Phosphohalohydrinverbindungen nach Anspruch 1 mit der Formel:

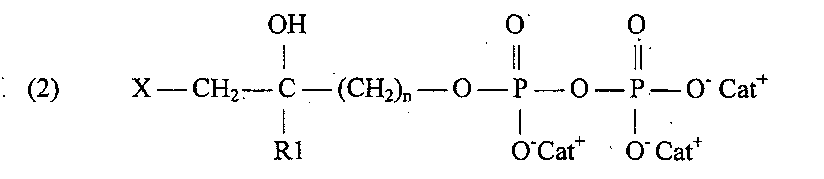

3. Phosphohalohydrinverbindungen nach Anspruch 1 mit der Formel:

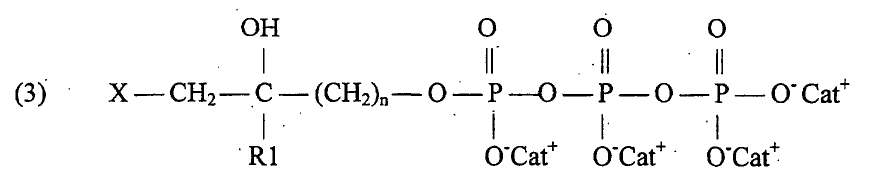

4. Phosphohalohydrinverbindungen nach Anspruch 1 mit der Formel: 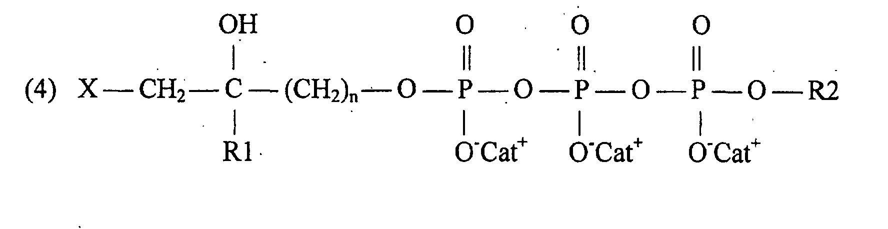 wobei R2 ein organischer oder mineralischer Substituent ist, der ausgewählt wurde aus der Gruppe bestehend aus Substituenten, die die Bildung der Halohydrinfunktion 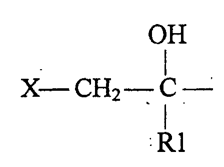 auf der Basis der Alkenfunktion 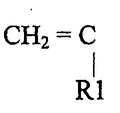 nicht behindern, und aus Wasserstoff X₂ in Anwesenheit von Wasser; und aus Substituenten, für die eine nichtreaktive Verbindung R2-O-Y mit der Halohydrinfunktion der Verbindung der Formel: 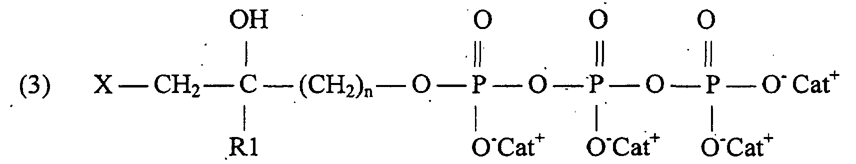 existiert, und die so gewählt wird, dass R2-O-Y auf dem Phosphatterminal dieser Verbindung (3) reagiert; so dass die Verbindung (4) entsteht.

5. Verbindungen nach einem der Ansprüche 1 und 4, ferner umfassend wenigstens eine Gruppierung ausgewählt aus der Gruppe bestehend aus Derivaten von Nukleosiden, Oligonukleotiden, Nukleinsäuren (ARN, ADN), Aminsäuren, Peptiden, Proteinen, Monosacchariden, Oligosacchariden, Polysacchariden, Fettsäuren, einfachen Lipiden, komplexen Lipiden, Folsäure, Tetrahydrofolsäure, Phosphorsäuren, Inositol, Vitaminen, Coenzymen, Flavenoiden, Aldehyden, Epoxiden und Halohydrinen.

6. Verbindungen nach Anspruch 4 und 5, bei denen R2 ausgewählt wird aus der Gruppe bestehend aus Derivaten von Nukleosiden, Oligonukleotiden, Nucleinsäuren (ARN, ADN), Aminsäuren, Peptiden, Proteinen, Monosacchariden, Oligosacchariden, Polysacchariden, Fettsäuren, einfachen Lipiden, komplexen Lipiden, Folsäure, Tetrahydrofolsäure, Phosphorsäuren, Inositol, Vitaminen, Coenzymen, Flavenoiden, Phosphohalohydrinen gemäß Formel (1), Aldehyden, Epoxiden und Halohydrinen.

7. Phosphohalohydrinverbindungen nach Anspruch 1 mit der Formel: 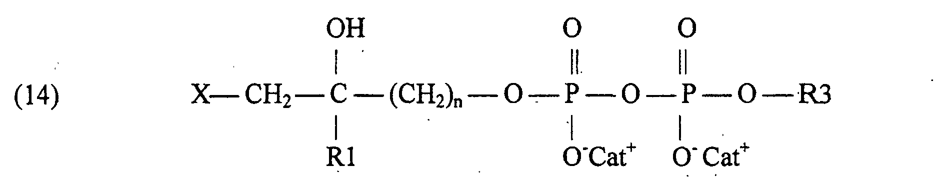 wobei
X ein Halogen ist, ausgewählt aus Iod, Brom und Chlor,
R1 aus -CH₃ und -CH₂-CH₃ ausgewählt ist,
Cat⁺ ein oder mehrere organische oder mineralische Katione (einschließlich dem Proton) repräsentiert, die in derselben Verbindung identisch oder unterschiedlich sind,
n eine ganze Zahl zwischen 2 und 20 ist,
R3- ausgewählt wird aus:
- einer Halohydringruppierung der Formel: 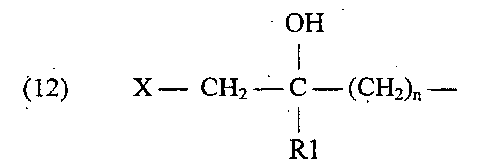
- einer Epoxidgruppierung der Formel: 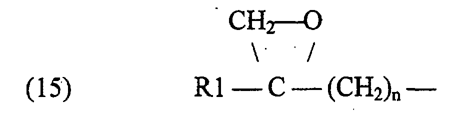
- einer Alkengruppierung der Formel: 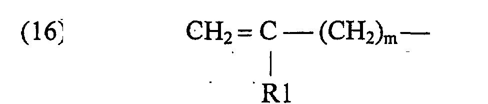
wobei m eine ganze Zahl zwischen 1 und 20 ist.

8. Verbindungen nach Anspruch 1 bis 7 für die Verwendung als Aktivator von Tγ9δ2-Lymphozyten.

9. Verbindungen nach Anspruch 1 bis 8 für die Verwendung als therapeutisch aktive Substanzen.

10. Verbindungen nach Anspruch 1 bis 9 für die Verwendung in einer immunstimulanten therapeutischen Zusammensetzung oder einem Vakzin für Primate.

11. Verbindungen nach Anspruch 1 bis 10 für die Verwendung als Antigene von Tγ9δ2-Lymphozyten in einer therapeutischen Zusammensetzung, insbesondere einer immunstimulanten Zusammensetzung oder einem Vakzin für Primate.

12. Verfahren zur Herstellung einer Verbindung, umfassend wenigstens eine Phosphohalohydringruppierung der Formel: 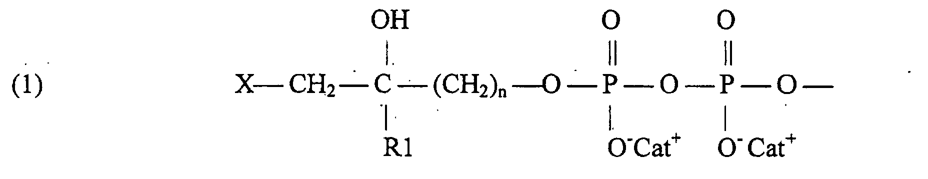 wobei
X ein Halogen ist, ausgewählt aus I, Br, Cl,
R1 aus ―CH₃ und -CH₂-CH₃ ausgewählt ist,
Cat⁺ ein organisches oder mineralisches Kation ist,
und n eine ganze Zahl zwischen 2 und 20 ist,
**dadurch gekennzeichnet, dass** das Halogen X₂ in Anwesenheit von Wasser mit einer Ausgangsverbindung reagieren gelassen wird, die wenigstens eine phosphatierte Alkengruppierung der folgenden Formel umfasst:

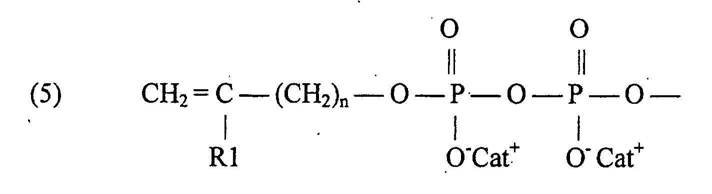

13. Verfahren nach Anspruch 12, **dadurch gekennzeichnet, dass** eine wässrige X₂-Halogenlösung in einem wässrigen oder hydroalkoholischen Milieu mit neutralem pH-Wert bei einer Temperatur von weniger als 30°C mit einem Salz reagieren gelassen wird, das aus der Ausgangsverbindung gebildet wurde.

14. Verfahren nach Anspruch 13, **dadurch gekennzeichnet, dass** die Reaktion unter atmosphärischem Druck bei einer Temperatur zwischen 0°C und 25°C stattfindet.

15. Verfahren nach einem der Ansprüche 12 bis 14, **dadurch gekennzeichnet, dass** die Ausgangsverbindung ein Salz der folgenden Formel ist:

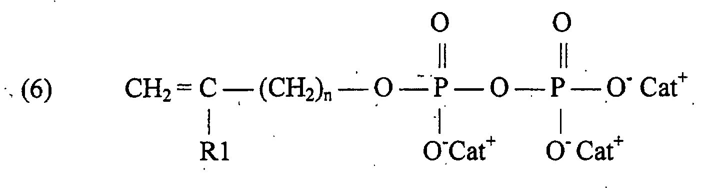

16. Verfahren nach einem der Ansprüche 12 bis 14, **dadurch gekennzeichnet, dass** die Ausgangsverbindung ein Salz der folgenden Formel ist:

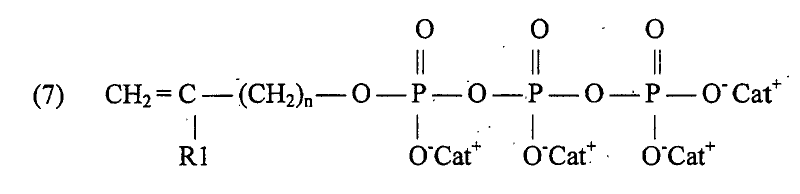

17. Verfahren nach Anspruch 16, **dadurch gekennzeichnet, dass** in einer letzten Stufe die erhaltene Verbindung : 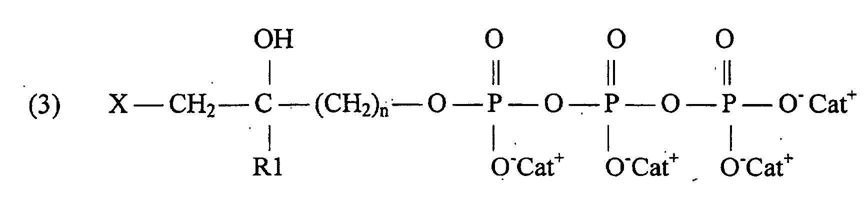 mit einer Verbindung R2-O-Y reagieren gelassen wird, wobei -O-Y eine Austrittsgruppierung und R2 ein organischer oder mineralischer Substituent ist, der so gewählt wird, dass R2-O-Y durch eine Reaktion mit Verbindung (3) die Verbindung der folgenden Formel bilden kann:

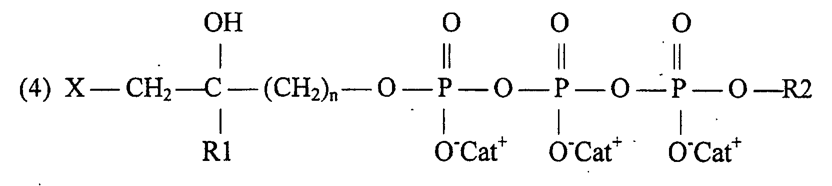

18. Verfahren nach einem der Ansprüche 12 bis 14, **dadurch gekennzeichnet, dass** die Ausgangsverbindung ein Salz der folgenden Formel ist: 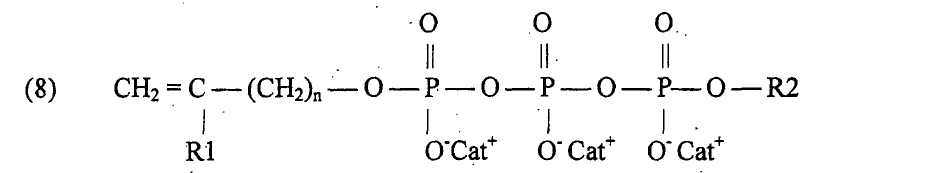 wobei R2 ein organischer oder mineralischer Substituent ist, dessen Aufgabe es ist, die Bildung der Halohydrinfunktion: 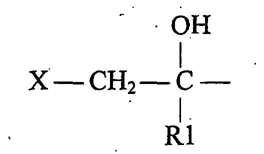 auf der Basis der Alkenfunktion 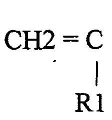 und des Halogens X₂ in Anwesenheit von Wasser nicht zu behindern.

19. Zusammensetzung für die extrakorporale Diagnostik, **dadurch gekennzeichnet, dass** sie wenigstens eine Verbindung nach einem der Ansprüche 1 bis 7 umfasst.

20. Therapeutische Zusammensetzung, **dadurch gekennzeichnet, dass** sie wenigstens eine Verbindung nach einem der Ansprüche 1 bis 7 umfasst.

21. Therapeutische Zusammensetzung, **dadurch gekennzeichnet, dass** sie eine einem Primaten verabreichbare Menge, insbesondere in Kontakt mit peripherem Blut oder über einen topischen Weg, von wenigstens einer Verbindung nach einem der Ansprüche 1 bis 7 umfasst.

22. Zusammensetzung nach einem der Ansprüche 19 bis 21, **dadurch gekennzeichnet, dass** sie darüber hinaus Tγ9δ2-Lymphozyten von Primaten umfasst.

23. Zusammensetzung nach einem der Ansprüche 19 bis 22, **dadurch gekennzeichnet, dass** sie ferner einen Interleukinanteil umfasst, der ein lymphozytäres Wachstum in einem Milieu erzeugen kann, in dem sie verabreicht werden soll.

24. Verfahren zur Herstellung einer Zusammensetzung mit der Eigenschaft, die Tγ9δ2-Lymphozyten zu aktivieren, in der wenigstens eine Verbindung nach einem der Ansprüche 1 bis 7 verwendet wird.

25. Verfahren zur Herstellung einer therapeutischen Zusammensetzung für eine präventive oder kurative Behandlung einer Pathologie, die für Tγ9δ2-Lymphozyten empfindliche pathogene Zellen erzeugt, bei dem wenigstens eine Verbindung nach einem der Ansprüche 1 bis 7 verwendet wird.

26. Verfahren zur Herstellung einer therapeutischen Zusammensetzung, die einem Primaten für die präventive oder kurative Behandlung einer Pathologie verabreicht werden soll, die für Tγ9δ2-Lymphozyten empfindliche Zellen erzeugt, bei dem wenigstens eine Verbindung nach einem der Ansprüche 1 bis 7 verwendet wird.

27. Verfahren zur Herstellung einer therapeutischen Zusammensetzung, die einem Primaten für die präventive oder kurative Behandlung einer Pathologie verabreicht werden soll, ausgewählt aus der Gruppe bestehend aus Krebs, Infektionskrankheiten, Parasitosen und Pathologien mit Immunmangelsyndrom, bei dem wenigstens eine Verbindung nach einem der Ansprüche 1 bis 7 verwendet wird.

28. Verfahren nach einem der Ansprüche 24 bis 27, bei dem wenigstens eine Verbindung nach einem der Ansprüche 1 bis 7 in Kontakt mit einem Tγ9δ2-Lymphozyten enthaltenden Milieu gebracht wird, das mit dem T-Lymphozytenwachstum kompatibel ist, in einer Menge, die ausreicht, um diese Tγ9δ2-Lymphozyten in diesem Milieu zu aktivieren.

29. Verfahren nach Anspruch 28, bei dem das genannte Milieu eine Substanz umfasst, die aus Primatenblut und Extrakten von Primatenblut ausgewählt wird.

30. Verfahren zur extrakorporalen Aktivierung von Tγ9δ2-Lymphozyten von Primaten, bei dem die Tγ9δ2-Lymphozyten in einem die mit T-Lymphozytenwachstum kompatiblen Tγ9δ2-Lymphozyten enthaltenden extrakorporalen Milieu mit wenigstens einer Verbindung nach einem der Ansprüche 1 bis 7 in Kontakt gebracht werden.

31. Verfahren nach Anspruch 30, bei dem wenigstens eine Verbindung nach einem der Ansprüche 1 bis 7 in dem Milieu in einer Konzentration verwendet wird, die eine Aktivierung der polyklonalen Proliferation von Tγ9δ2-Lymphozyten bewirkt.

32. Verfahren nach einem der Ansprüche 30 bis 31, bei dem in das Milieu ein Interleukinanteil geleitet wird, der ausreicht, um ein lymphozytäres Wachstum in dem Milieu zu erzeugen.

## Claims

1. Compounds comprising at least one phosphohalohydrin group of the formula: 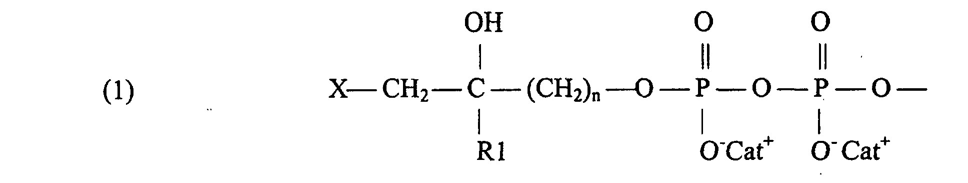 where
X is a halogen selected from among I, Br, Cl,
R1 is selected from among ―CH₃ and ―CH₂―CH₃,
Cat+ is an organic or inorganic cation,
and n is an integer between 2 and 20.

2. The phosphohalohydrin compounds as claimed in claim 1 of the formula:

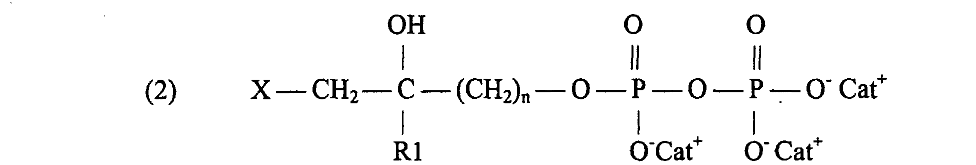

3. The phosphohalohydrin compounds as claimed in claim 1 of the formula:

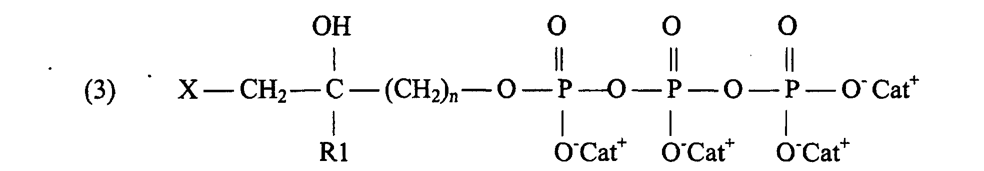

4. The phosphohalohydrin compounds as claimed in claim 1 of the formula: 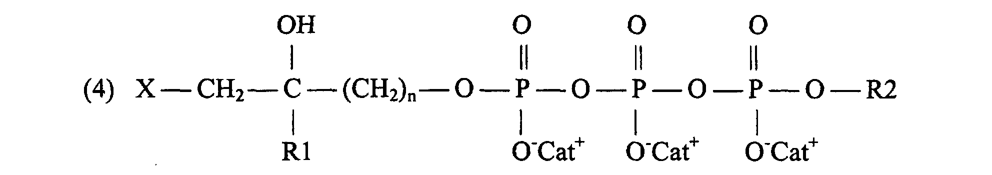 where R2 is an organic or inorganic substituent selected from the group comprising substituents which do not prevent formation of the halohydrin function 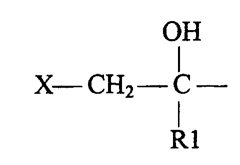 starting from the alkene function 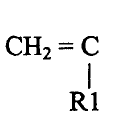 and halogen X₂ in the presence of water; and substituents for which there is an R2-O-Y compound which is not reactive towards the halohydrin function of the compound of the formula: 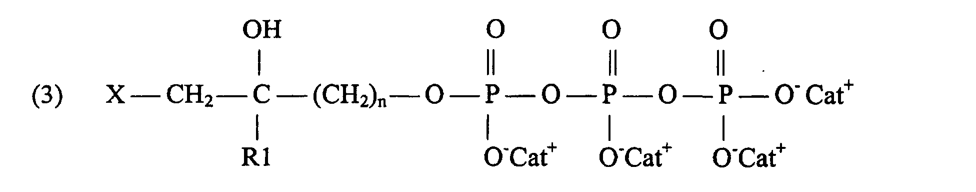 and selected such that R2-O-Y may react with the terminal phosphate of this compound (3) in order to obtain the compound (4).

5. The compounds as claimed in one of claims 1 and 4, moreover comprising at least one group selected from among the group comprising nucleoside derivatives, oligonucleotides, nucleic acids (RNA, DNA), amino acids, peptides, proteins, monosaccharides, oligosaccharides, polysaccharides, fatty acids, simple lipids, complex lipids, folic acid, tetrahydrofolic acid, phosphoric acids, inositol, vitamins, co-enzymes, flavonoids, aldehydes, epoxides and halohydrins.

6. The compounds as claimed in one of claims 4 and 5, in which R2 is selected from among the group comprising nucleoside derivatives, oligonucleotides, nucleic acids (RNA, DNA), amino acids, peptides, proteins, monosaccharides, oligosaccharides, polysaccharides, fatty acids, simple lipids, complex lipids, folic acid, tetrahydrofolic acid, phosphoric acids, inositol, vitamins, co-enzymes, flavonoids, phosphohalohydrins of the formula (I), aldehydes, epoxides and halohydrins.

7. The phosphohalohydrin compounds as claimed in claim 1 of the formula: 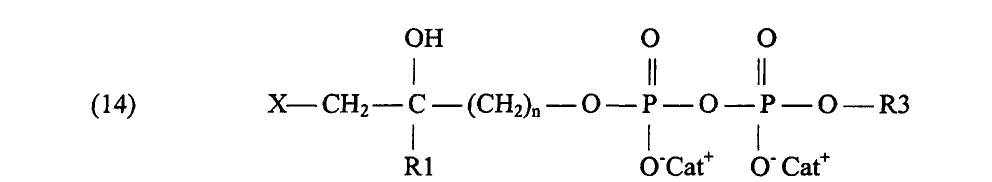 where
X is a halogen selected from among iodine, bromine and chlorine,
R1 is selected from among ―CH₃ and ―CH₂―CH₃,
Cat⁺ represents one or more organic or inorganic cation(s) (including the proton), which may be identical or different in the same compound,
n is an integer between 2 and 20,
R3- is selected from among:
- a halohydrin group of the formula: 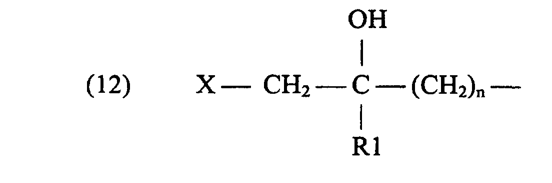
- an epoxide group of the formula: 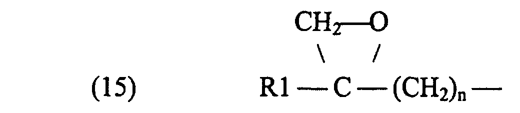
- an alkene group of the formula: 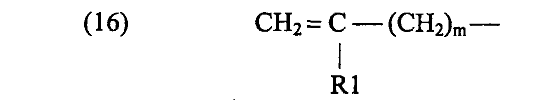
m being an integer between 1 and 20.

8. The compounds as claimed in one of claims 1 to 7 for the use thereof as a Tγ9δ2 lymphocyte activator.

9. The compounds as claimed in one of claims 1 to 8 for the use thereof as therapeutically active substances.

10. The compounds as claimed in one of claims 1 to 9 for the use thereof in an immunostimulant therapeutic composition or a vaccine for primates.

11. The compounds as claimed in one of claims 1 to 10 for the use thereof as Tγ9δ2 lymphocyte antigens in a therapeutic composition, in particular an immunostimulant therapeutic composition or a vaccine, for primates.

12. A process for the production of a compound comprising at least one phosphohalohydrin group of the formula: 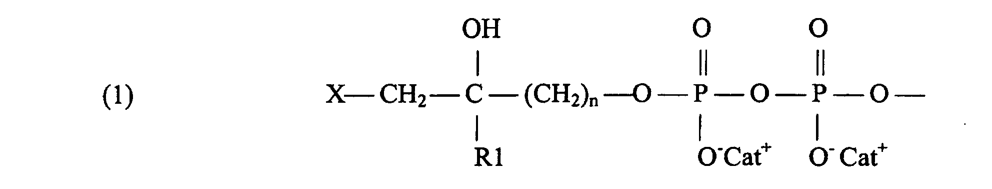 where
X is a halogen selected from among I, Br, Cl,
R1 is selected from among ―CH₃ and ―CH₂―CH₃,
Cat⁺ is an organic or inorganic cation,
and n is an integer between 2 and 20,
**characterized in that** the halogen X₂ is reacted in the presence of water with a starting compound comprising at least one phosphorylated alkene group of the formula:

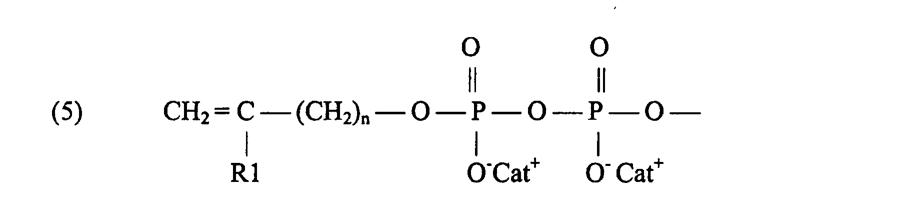

13. The process as claimed in claim 12, **characterized in that** a salt formed from the starting compound is reacted in an aqueous or aqueous/alcoholic medium, at neutral pH, at a temperature of below 30°C, with an aqueous solution of the halogen X₂.

14. The process as claimed in claim 13, **characterized in that** the reaction is performed at atmospheric temperature at a temperature of between 0°C and 25°C.

15. The process as claimed in one of claims 12 to 14, **characterized in that** the starting compound is a salt of the formula:

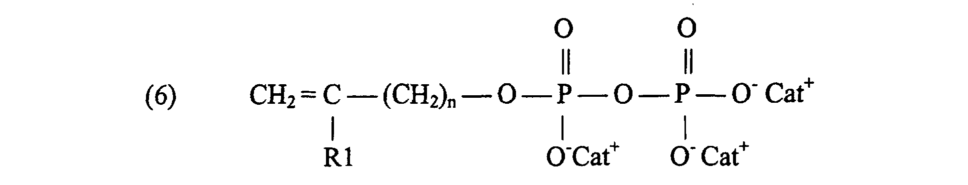

16. The process as claimed in one of claims 12 to 14, **characterized in that** the starting compound is a salt of the formula:

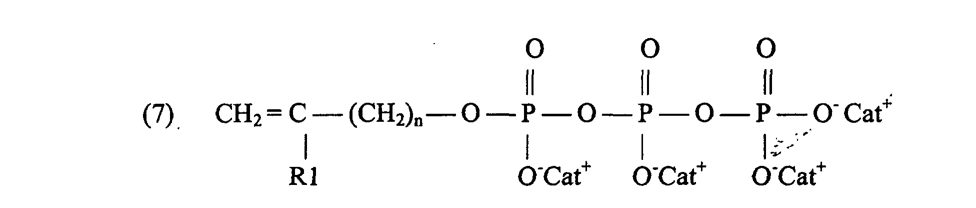

17. The process as claimed in claim 16, **characterized in that**, in a subsequent stage, the compound obtained: 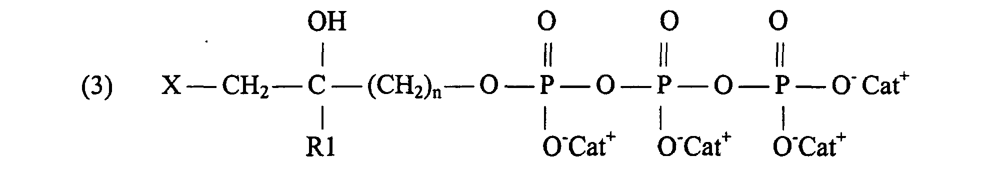 is reacted with a compound R2-O-Y, where -O-Y is a leaving group and R2 is an organic or inorganic substituent selected such that R2-O-Y is capable of forming, by reaction with the compound (3), the compound of the formula:

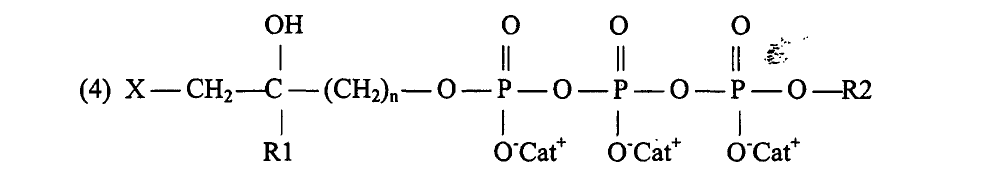

18. The process as claimed in one of claims 12 to 14, **characterized in that** the starting compound is a salt of the formula: 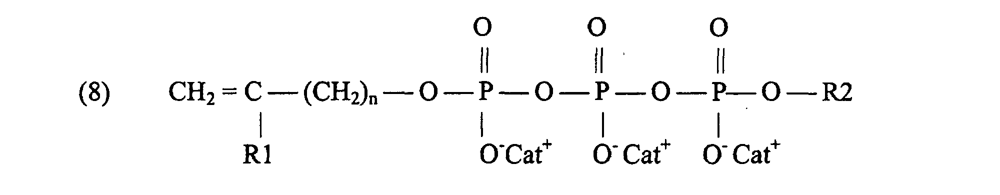 where R2 is an organic or inorganic substituent of a nature such as not to prevent formation of the halohydrin function 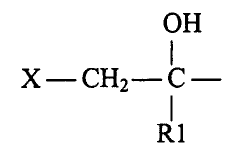 starting from the alkene function 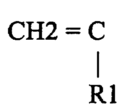 and halogen X₂ in the presence of water.

19. A composition for extracorporeal diagnostics, **characterized in that** it comprises at least one compound as claimed in one of claims 1 to 7.

20. A therapeutic composition, **characterized in that** it comprises at least one compound as claimed in one of claims 1 to 7.

21. A therapeutic composition, **characterized in that** it comprises a quantity capable of being administered to a primate, in particular in contact with the peripheral bloodstream or topically, of at least one compound as claimed in one of claims 1 to 7.

22. The composition as claimed in one of claims 19 to 21, **characterized in that** it moreover comprises primate Tγ9δ2 lymphocytes.

23. The composition as claimed in one of claims 19 to 22, **characterized in that** it moreover comprises a proportion of interleukin suitable to bring about lymphocyte growth in the medium into which it is to be administered.

24. A process for the production of a composition having the characteristic of activating Tγ9δ2 lymphocytes, in which process at least one compound as claimed in one of claims 1 to 7 is used.

25. A process for the production of a therapeutic composition intended for the preventive or curative treatment of a pathological condition which produces pathogenic cells sensitive to Tγ9δ2 lymphocytes, in which process at least one compound as claimed in one of claims 1 to 7 is used.

26. A process for the production of a therapeutic composition intended to be administered to a primate for the preventive or curative treatment of a pathological condition which produces cells sensitive to Tγ9δ2 lymphocytes, in which process at least one compound as claimed in one of claims 1 to 7 is used.

27. A process for the production of a therapeutic composition intended to be administered to a primate for the preventive or curative treatment of a pathological condition selected from among the group comprising cancers, infectious diseases, parasitic conditions, and pathological immunodeficiency syndromes, in which process at least one compound as claimed in one of claims 1 to 7 is used.

28. A process according to claim one of claims 24 to 27, in which at least one compound as claimed in one of claims 1 to 7 is brought into contact with a medium which contains Tγ9δ2 lymphocytes, and is compatible with T lymphocyte growth, in a quantity suitable for activating these Tγ9δ2 lymphocytes in this medium.

29. A process as claimed in claim 28, in which said medium comprises a substance selected from among primate blood and primate blood extracts.

30. A process for the extracorporeal activation of Tγ9δ2 lymphocytes, in which the Tγ9δ2 lymphocytes are brought into contact with at least one compound as claimed in one of claims 1 to 7 in an extracorporeal medium which contains Tγ9δ2 lymphocytes and is compatible with T lymphocyte growth.

31. The process as claimed in claim 30, in which at least one compound as claimed in one of claims 1 to 7 is used at a concentration in the medium which brings about activation of polyclonal proliferation of Tγ9δ2 lymphocytes.

32. The process as claimed in one of claims 30 to 31, in which a proportion of interleukin suitable to bring about lymphocyte growth in the medium is introduced into the medium.
